# EUROPEAN PATENT SPECIFICATION

(11) **EP 4 153 194 B1**
(45) Date of publication and mention of the grant of the patent: **18.02.2026**
(21) Application number: 21726393.8
(22) Date of filing: 18.05.2021
(51) Int. Cl.: A61K 35/17, A61K 38/47, A61K 38/48, A61P 31/00, A61P 35/00, A61P 37/00

(54) **METHODS FOR ENHANCING ADOPTIVE CELL TRANSFER IMMUNOTHERAPIES**
VERFAHREN ZUR VERBESSERUNG VON ADOPTIVEN ZELLTRANSFERIMMUNTHERAPIEN
MÉTHODES D'AMÉLIORATION D'IMMUNOTHÉRAPIES PAR TRANSFERT ADOPTIF DE CELLULES

(30) Priority: 19.05.2020 GB 202007434
(43) Date of publication of application: 29.03.2023
(73) Proprietor: Hansa Biopharma AB, 220 07 Lund (SE)
(72) Inventor: KJELLMAN, Christian, 220 07 Lund (SE)
(74) Representative: J A Kemp LLP
(86) International application number: PCT/EP2021/063164
(87) International publication number: WO 2021/233924

(56) References cited:
- WO-A2-2006/131347
- WO-A2-2008/071418
- WO-A2-2016/012285
- C. H. J. LAMERS ET AL: "Immune responses to transgene and retroviral vector in patients treated with ex vivo-engineered T cells", BLOOD, vol. 117, no. 1, 6 January 2011 (2011-01-06), US, pages 72 - 82, XP055234814, ISSN: 0006-4971, DOI: 10.1182/blood-2010-07-294520
- GOROVITS: "Immunogenicity of Chimeric Antigen Receptor T-Cell Therapeutics", 8 May 2019 (2019-05-08), XP055829951, Retrieved from the Internet <URL:http://www.ncbi.nlm.nih.gov/pubmed/31069709> [retrieved on 20210803]
- JIALI CHENG ET AL: "Understanding the Mechanisms of Resistance to CAR T-Cell Therapy in Malignancies", FRONTIERS IN ONCOLOGY, vol. 9, 21 November 2019 (2019-11-21), CH, XP055753227, ISSN: 2234-943X, DOI: 10.3389/fonc.2019.01237

## Description

### Field of the Invention

The present invention relates to improvements to adoptive cell transfer immunotherapies, as defined in the claims.

### Background of the Invention

Adoptive cell transfer immunotherapies are a newly established class of therapies for treating various diseases, including cancer, autoimmune conditions and infections. For the treatment of solid tumors or tumors of hematogenic origin, patients' T-cells have been expanded *in vitro,* ideally specific for tumor-associated antigens, before reinfusion. T-cell numbers, specificity and efficacy have been limiting factors of these treatments. One exemplary factor for tumor escape is the down-regulation of HLAs needed to present tumor antigens.

Chimeric antigen receptor (CAR) transfected cells are an emerging field of cell-based immune therapies making use of the innate cytolytic potential of the patients' own NK and T-cells. These modified autologous cells can be directed against B-cell malignancies or solid tumors like colon or breast cancer through the introduction of cell-surface expressed chimeric antigen receptors (CARs) receiving their specificity from tumor specific scFv domains. Chimeric antigen receptor (CAR) expressing T-cells combine the anti-tumour activity of cytotoxic T-cells with the specificity and affinity of scFv elements derived from tumor-associated antigen-specific antibodies. Autologous T-cells can be harvested from the patients in sufficient numbers before *in vitro* transfection with the CAR of choice. Further expansion provides high numbers of HLA-independent tumor-specific cytotoxic T-cells.

The efficacy of CAR T-cell (CAR-T) therapies can be limited by the survival and sustained activity of the CAR T-cells in the patient after injection. However, the factors that limit the survival and efficacy of CAR T-cells remain poorly examined and controversial. Even though humoral responses against CAR T-cells can theoretically elicit antibody-mediated effector mechanisms, little direct evidence has been generated to show that the limiting factors for CAR T-cell success are caused by antibodies. Instead, tumor escape due to loss of target antigens like CD19 (Majzner and Mackall, 2018, Cancer Discov., 8(10):1219-26), CAR T-cells lacking polyfunctionality (Rossi et al., 2018, Blood, 132(8):804-814), or lack of tumor infiltration (Newick et al., 2017, Annu. Rev. Med. 68:139-152) are a concern for the success of CAR T-cells. Failure of repeat infusion has often been attributed to T-cell mediated cytotoxicity caused by the presentation of mouse peptides, for example, by the scFv on the HLA of CAR T-cells (Turtle et al., -2016, J. Clin. Invest., 126(6):2123-2138). In line with this, the FDA approved CAR T cell tisagenlecleucel (Kymriah) is said not to be impacted by preexisting and induced humoral immunity (Thudium-Muller, J. Clin. Oncology, 36(15), 2018).

The immunoglobulin G-degrading cysteine protease, imlifidase (IdeS) is an IgG endopeptidase that is currently under development as a rapid desensitization treatment in kidney transplantation. Imlifidase is highly specific and cleaves all subclasses of human IgG. Imlifidase may be useful for reducing competition for Fc-receptors when administering antibody drug products (WO 2016/012285).

There is a requirement for improved methods of treating cancer, autoimmune conditions, infections and conditions mediated by B-cells expressing deleterious antibodies.

### Summary of the Invention

The invention provides methods of improving the benefit to a patient of an adoptive cell transfer immunotherapy comprising administering a protein that has IgG cysteine protease or IgG endoglycosidase activity in combination with the adoptive cell transfer immunotherapy, as defined in the claims. The inventors have identified that the efficacy of adoptive cell transfer immunotherapies may be reduced by the limited survival and sustained activity of the transferred cells, such as CAR-T cells, and the inventors have shown in the examples that proteins with IgG cysteine protease or IgG endoglycosidase activity may protect transferred cells. In particular, the inventors have identified that cell surface receptor-specific antibodies, including pre-existing antibodies and antibodies generated after dosing with transferred cells, may cut short the potential of transferred cells and that the therapeutic effect of the transferred cells will profit from the removal of antibody effector functions through the conditioning of the recipient. Therefore, administering proteins with IgG cysteine protease or IgG endoglycosidase activity may increase the survival and activity of transferred cells and provide improved adoptive cell transfer immunotherapy treatments. The inventors have also identified that cell surface receptor-specific antibodies, for example against receptor constructs of CAR-T cells and other cell-based therapeutics, can interfere with the interaction between the receptor and its target protein, and that proteins with IgG cysteine protease or IgG endoglycosidase activity are effective for digesting the antibodies and increasing the binding of the receptor to its target.

As demonstrated in the examples, treatment with imlifidase (IdeS), an IgG cysteine protease, and EndoS, an IgG endoglycosidase, mitigates some of the limiting effector functions of the antibodies directed against CAR-T cells and other cell-based therapeutics. Therefore, the invention provides methods of improving the benefit to a patient of an adoptive cell transfer immunotherapy comprising administering a protein that has IgG cysteine protease or IgG endoglycosidase activity in combination with an adoptive cell transfer immunotherapy. In preferred embodiments, the invention provides a protein that has IgG cysteine protease or IgG endoglycosidase activity for use in a method for treating cancer, wherein the protein that has IgG cysteine protease or IgG endoglycosidase activity is used in combination with an adoptive cell transfer immunotherapy.

One limiting factor of CAR-T therapy and other adoptive cell transfer immunotherapies might be naturally occurring pre-existing antibodies against the CAR constructs affecting their efficacy through different antibody-mediated effector mechanisms like complement-dependent cytotoxicity (CDC), antibody-dependent cellular phagocytosis (ADCP), antibody-dependent cellular cytotoxicity (ADCC), exhaustion due to tonic stimulation or receptor activation-induced cell death. Survival and sustained activity of the CAR T-cells in the patient may be reduced by such antibodies after injection. Infused CAR-T cells might even induce increased antibody levels against the chimeric receptor and thereby prevent the interaction of the CARs with their target cells during the course of the first treatment as well as limiting their expansion and persistence. The success of a second treatment round could be even more challenging due to increased and probably higher affinity anti-drug antibody (ADA) levels. Even though CAR T-cells are of autologous origin, changes introduced by the chimeric receptors and expression of virus antigens from the T-cell transfection process make them vulnerable for host immune responses. Some immunogenic parts can be junctional regions between receptor components but most prominently the scFv part, which in the early stages of CAR-T development were taken from tumor-specific mouse IgG, for example. Even though subsequent CAR constructs often use humanized IgGs to reduce the number of foreign epitopes, these scFv still contain neo-epitopes in the antigen-binding domain. It has been shown that these foreign epitopes can be antigens for cellular host T-cell responses, limiting the survival of the CAR T-cells (Harding et al., 2010, MAbs, 2(3):256-65, Meunier et al., 2019, Cell. & Mol. Immunology). In contrast, the data in the examples demonstrate that proteins with IgG cysteine protease or IgG endoglycosidase activity may increase the survival and activity of transferred cells and provide improved treatment through inactivation of cell surface receptor-specific antibodies.

There are different possible mechanisms by which the activity, expansion, and survival of transferred cells such as CAR T-cells could be affected by antibodies. For example, CAR-specific antibodies could facilitate the destruction of CAR T-cells by means of complement deposition CDC and/or ADCP. The examples demonstrate that proteins with IgG cysteine protease or IgG endoglycosidase activity may be effective at mitigating these processes and thereby improving adoptive cell transfer immunotherapies. The binding of antibodies themselves to the receptor might also lead to ADCC, exhaustion or receptor-activation induced cell death. Proteins with IgG cysteine protease or IgG endoglycosidase activity will also be useful for mitigating these processes.

In light of these developments, the invention provides a protein that has IgG cysteine protease or IgG endoglycosidase activity for use in medicine, wherein the protein that has IgG cysteine protease or IgG endoglycosidase activity is used in combination with an adoptive cell transfer immunotherapy. In certain embodiments, the protein is administered prior to the adoptive cell transfer immunotherapy. The examples demonstrate that IdeS and EndoS are effective for inactivating pre-existing antibodies, for example IdeS and EndoS were effective when used before addition of a complement source or effector cells. The examples also demonstrate that antibodies present in the serum of healthy individuals and HLA-sensitized patients that have not been administered an adoptive cell transfer immunotherapy are able to bind receptor constructs and cells such as CAR T-cells and mediate deleterious effects such as ADCP, ADCC and interfering with target binding (such allogenic antibodies may be induced by pregnancy or blood transfusion, for example), all of which can be reduced by IdeS treatment. In preferred embodiments, the protein is administered after an administration of the adoptive cell transfer immunotherapy. The examples demonstrate that IdeS and EndoS are effective for inactivating induced antibodies, because immune thrombocytopenia was reduced when Ides and EndoS were administered after the anti-platelet specific antibodies. The examples also demonstrate that allogenic antibodies present in human sera can negatively affect adoptive cell transfer immunotherapy cells such as CAR-T cells, and that the proteins of the invention can reduce or prevent such negative effects. Adoptive cell transfer immunotherapy may induce such allogenic antibodies, so the proteins of the invention may be useful when administered after an administration of the adoptive cell transfer immunotherapy.

The disclosure also provides methods of improving the benefit to a patient of an adoptive cell transfer immunotherapy, of prolonging the survival and/or enhancing the proliferation of cells administered as part of an adoptive cell transfer, of conditioning or preparing a patient for an adoptive cell transfer immunotherapy, of reducing serum IgG levels or reducing complement and/or Fc receptor binding by serum IgG molecules in a patient undergoing or scheduled to undergo an adoptive cell transfer immunotherapy, wherein the methods comprise administering a protein that has IgG cysteine protease or IgG endoglycosidase activity. The effects shown in the examples for the polypeptides of the invention will provide significant benefits in such methods.

The disclosure also provides methods for increasing the potency of an adoptive cell transfer therapy or increasing the binding between the cell surface receptor of an adoptive cell transfer therapy and its target, comprising administering a protein that has IgG cysteine protease or IgG endoglycosidase activity prior to, subsequent to or concurrently with the adoptive cell transfer immunotherapy. The examples demonstrate that the polypeptides of the invention are effective for increasing such potency and binding. In preferred such embodiments, the target is CD19, and preferably the cell surface receptor is an anti-CD19 CAR.

In certain embodiments of the invention, the protein that has IgG cysteine protease or IgG endoglycosidase activity improves the benefit to a patient of an adoptive cell transfer immunotherapy or improves the treatment of cancer, an autoimmune condition, a condition mediated by B-cells producing deleterious antibodies, or an infection, by removing IgG antibodies that inhibit the binding of the cell surface receptor of an adoptive cell transfer therapy to its target. Said antibodies may bind to the cell surface receptor, in particular a CAR, or may bind to a CAR-adaptor molecule, or may bind to the target itself, and may sterically hinder binding between the receptor and its target. In certain embodiments, the protein of the invention increases clearance of antibody fragments, in particular F(ab')₂ fragments, that block the interaction between the target and the cell surface receptor. In preferred embodiments, the target is CD19, and preferably the cell surface receptor is an anti-CD19 CAR.

In preferred embodiments, the method of the invention uses an IgG cysteine protease. In especially preferred embodiments, the IgG cysteine protease is an IdeS or IdeZ polypeptide, most preferably an IdeS polypeptide, such as a polypeptide having a sequence that is at least 80% identical to SEQ ID NO: 2, 4 or 5, such as at least 85%, 90%, 95% or 99% identical. The examples demonstrate that such polypeptides are effective for protecting cells.

In further embodiments, the IgG endoglycosidase is an EndoS polypeptide, such as a polypeptide having a sequence that is at least 80% identical to SEQ ID NO: 90, such as at least 85%, 90%, 95% or 99% identical. The examples demonstrate that such polypeptides are effective for protecting cells.

In especially preferred embodiments of the invention, the method comprises administering an IdeS polypeptide in combination with a CAR-T therapy, preferably in the treatment of cancer.

In especially preferred embodiments, the methods of the invention comprise administering an IdeS polypeptide after an adoptive cell immunotherapy, such as administering an IdeS polypeptide to a patient that has previously received an adoptive cell immunotherapy. Such methods will reduce the effects of ADA induced by the cell therapy and will improve survival of the cells. In further especially preferred embodiments, an IdeS polypeptide will be administered to a patient that has previously received an adoptive cell immunotherapy and is scheduled to receive an additional adoptive cell immunotherapy, such as with the same or similar construct.

The invention also provides compositions, in particular pharmaceutical compositions, comprising a protein that has IgG cysteine protease or IgG endoglycosidase activity, for use in the methods of the invention.

### Brief Description of the Figures

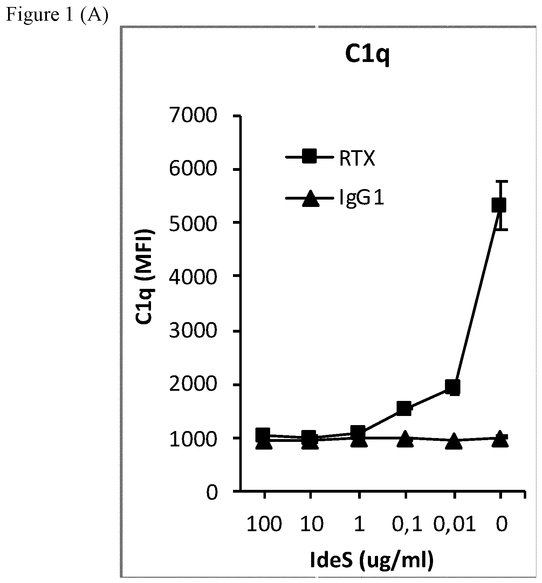

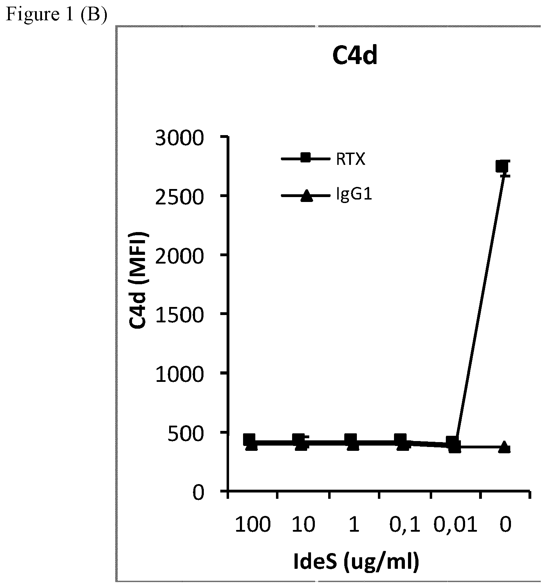

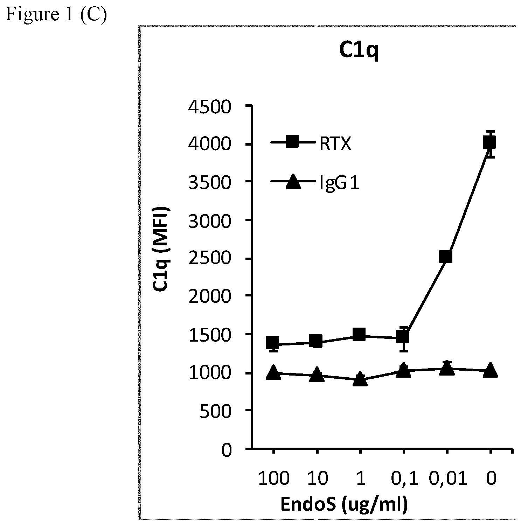

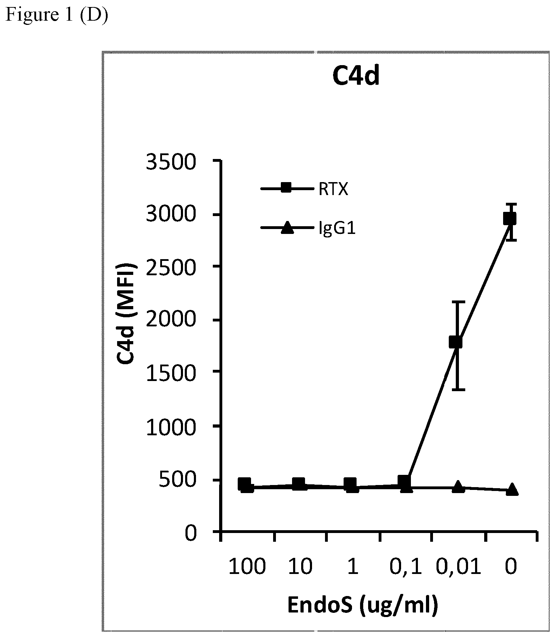

**Figure 1****: C1q and C4d deposition of antibody-targeted Daudi cells can be averted by IdeS or EndoS treatment.** Daudi cells were incubated with RTX (or IgG1 isotype control) together with titrations of IdeS (A+B) or EndoS (C+D) for approx. 2h before adding human serum complement for an additional 2 hours. The cells were aliquoted and stained with anti-human C1q (A+C) or C4d (B+D) followed by a final detection step with SA-PE. The cells were analysed in FL2 using an Accuri C6 flow cytometer. MFI values are depicted.

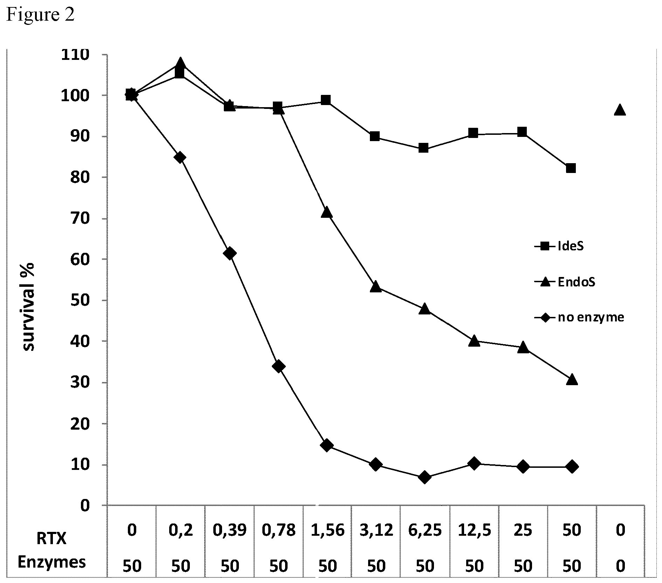

**Figure 2****: ADCC can be prevented or ameliorated with IdeS or EndoS.** CD20-positive Daudi cells were incubated with RTX (titrated from 0,2 to 50 µg/ml) together with either 50 µg/ml IdeS (square), EndoS (triangle), or medium (diamond). CDC was induced through the addition of baby rabbit serum as complement source. Results were normalized as percent cell survival without RTX set as 100% after 90 min incubation. Cell cytotoxicity was assessed using the cell counting kit-8 (CCK8). The normal positive control group did not receive enzymes for treatment but only titrated RTX, which represent maximum toxicity of RTX at the indicated concentrations.

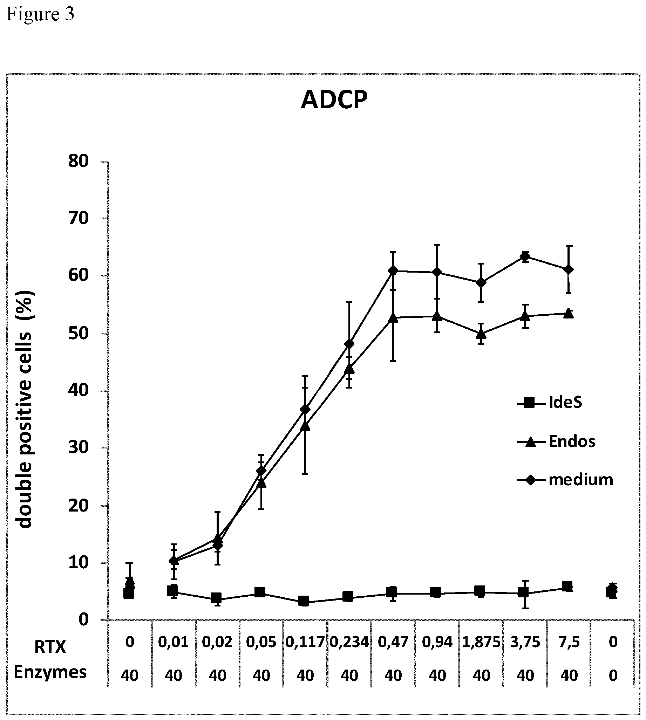

**Figure 3****: IdeS treatment of opsonized target cells prevents ADCP.** Calcein stained Daudi cells were opsonized with titrated RTX (7,5 down to 0,01 µg/ml) which were incubated with 40 µg/ml of either IdeS (square), EndoS (triangle) or no enzyme (medium only, diamond) before adding FarRed stained THP1 effector cells. Cells were fixed after 2h incubation and analysed by flow cytometer in FL2 and FL4. The gated FL2 positive cells were set as 100%. This population was further divided into FL2 single and FL2-FL4 double positive cells, which were a read-out for THP1-phagocytosed Daudi cells.

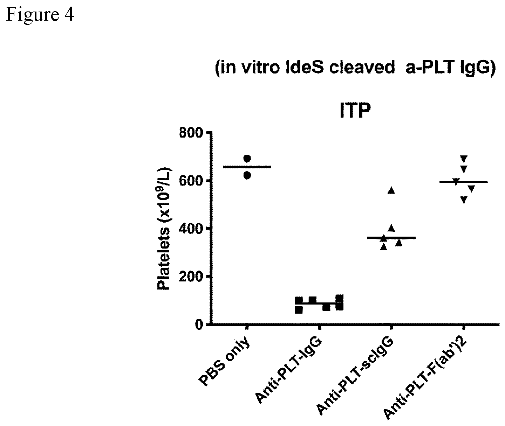

**Figure 4****: *In vitro* IdeS-cleaved anti-PLT antibodies do not induce ITP.** Thrombocytopenic anti-PLT IgG (250 µg/mouse) induced ITP in BALB/c mice after a single injection (squares). Single cleaved anti-PLT IgG (triangle) only partly induced ITP at the same dose, while fully cleaved anti-PLT IgG into F(ab')2 and Fc did not affect the normal platelet levels. Mice received 0.25 mg/mouse of purified IgG intraperitoneally in 200 µl PBS. Platelet counts were determined on day 1 using an automatic cell counter, VetScan HM5.

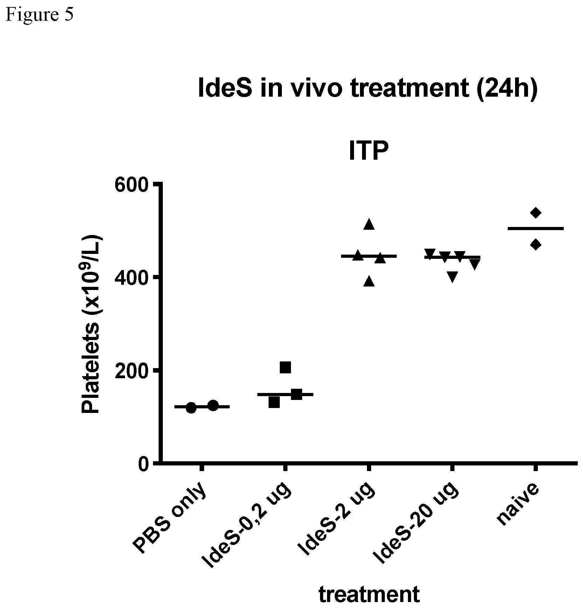

**Figure 5****: Antibody-induced ITP can be prevented with *in vivo* IdeS treatment.** BALB/c mice were primed for ITP by a single i.p. injection of purified intact rabbit anti-mouse thrombocyte IgG (0.25 mg/mouse). One hour after the induction of ITP, treatment with IdeS was administered i.v. at three different doses (0.2, 2 and 20 µg/mouse). The "PBS only" (circles) treatment group received no IdeS but only anti-PLT-IgG as positive control for ITP induction. Naïve mice (diamonds) received only carrier solutions represent the health control group. Platelet counts were determined on day 1 using an automatic cell counter, VetScan HM5.

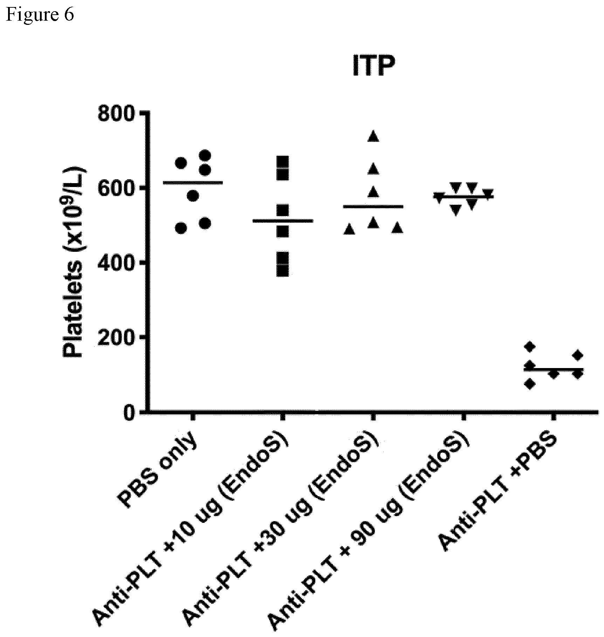

**Figure 6****: EndoS prevents ITP in mice even post anti-PLT antibody injection.** BALB/c mice (n=6 per group) were injected with 50 µg anti-PLT IgG to prime for ITP, or with PBS only (circle) as control for normal levels of platelets. 30 min later therapeutic i.p. injections with EndoS (10, 30 or 90 µg/mouse) were injected. The ITP-induction control was injected with PBS (diamonds). Blood was drawn after 24hrs and analyzed for platelet counts using an automatic cell counter, VetScan HM5.

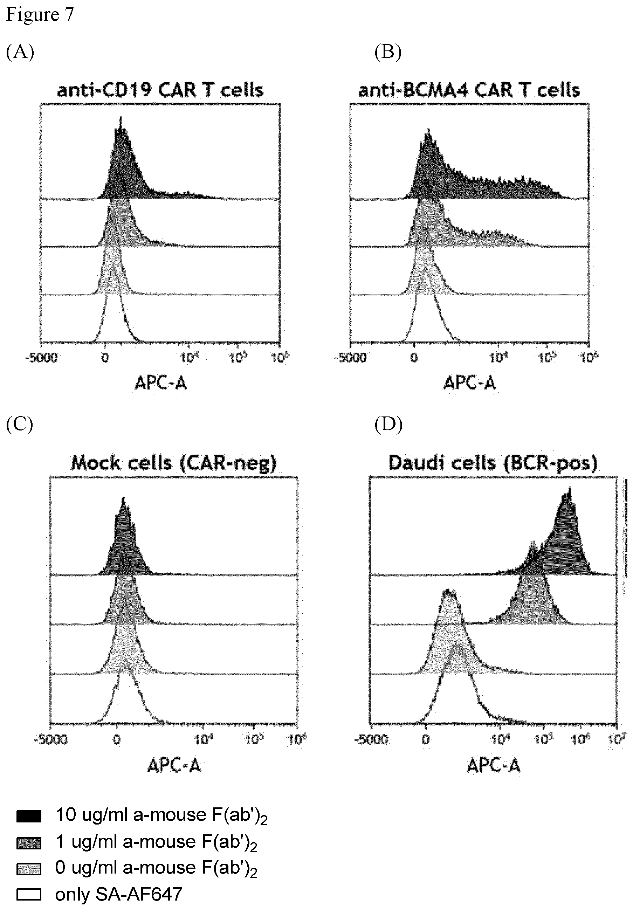

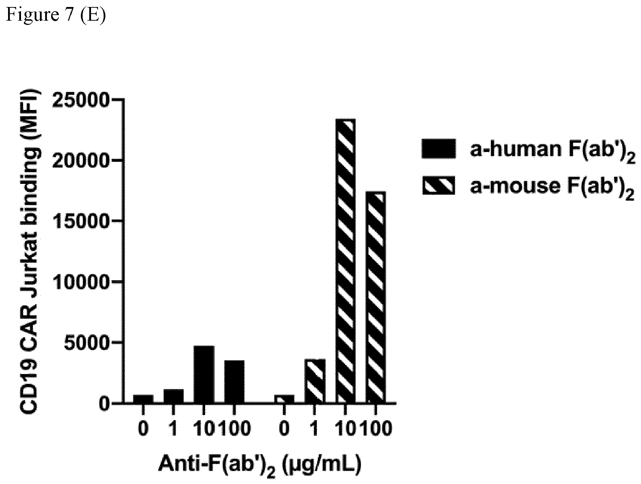

**Figure 7****: Identification of anti-CAR specific antibodies - F(ab')2-specific polyclonal antibodies bind specifically to CAR T-cell receptors.** Polyclonal rabbit anti-mouse F(ab')2 antibodies (10, 1 µg/mL) were evaluated for binding to primary CAR T-cells, including (A) anti-CD19 CAR T-cells, and (B) anti-BCMA4 CAR T-cells, and (C) mock-transfected T-cells, and (D) BCR expressing Daudi cells as negative and positive controls, respectively. Bound anti-F(ab')2 antibodies were detected by flow cytometry analysis with biotinylated anti-rabbit Fc and SA-AF647. Similarly, binding of polyclonal rabbit anti-mouse F(ab')2 and anti-human F(ab')2 (10 µg/mL) was also assessed using an anti-CD19 CAR-Jurkat T-cell line (E) (CARJ-ZP005, Creative biolabs) by FACS analysis, expressed as MFI.

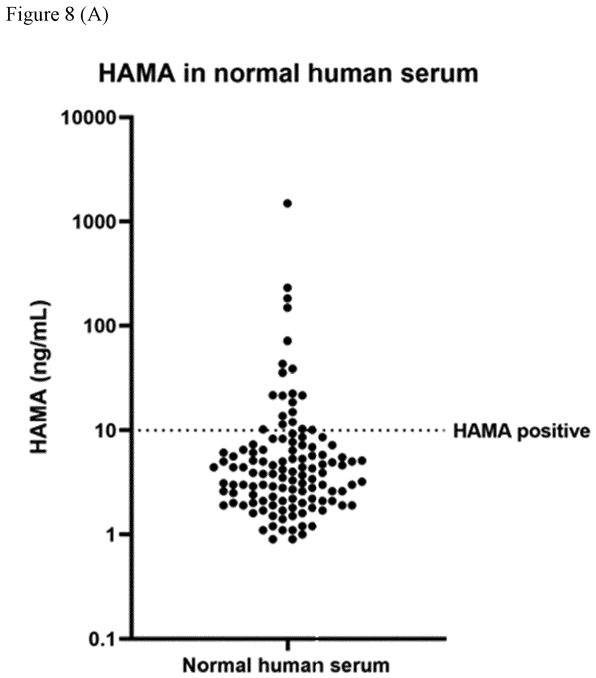

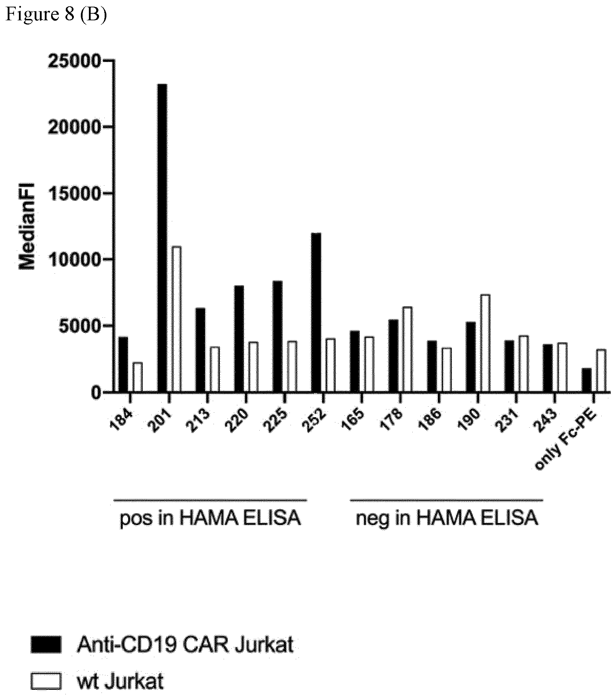

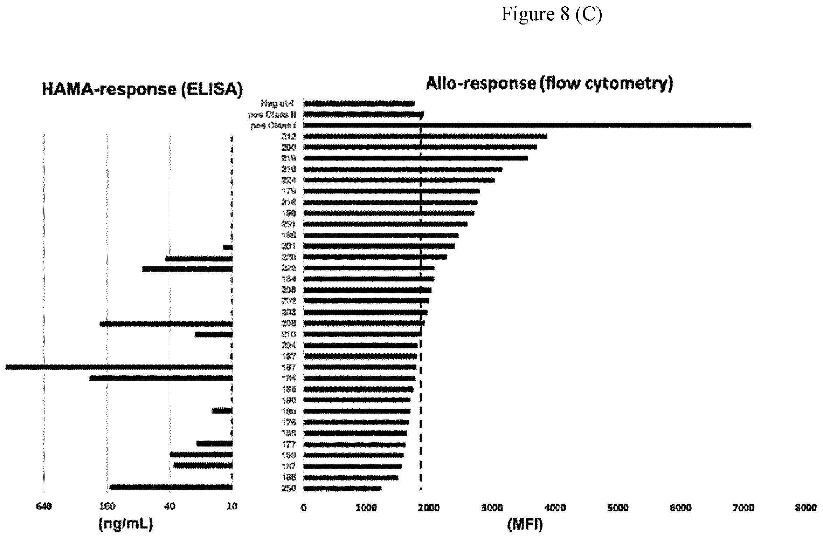

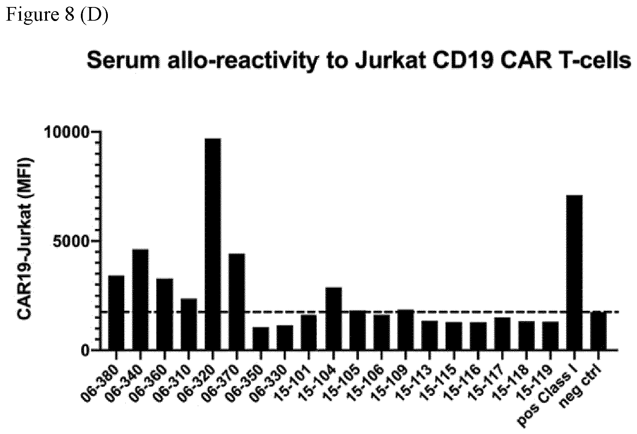

**Figure 8****: Identification of HAMA and anti-CD19 CAR Jurkat T-cell allo-specific sera.** (A) Normal human serum samples (BioIVT) were screened for human anti-mouse IgG antibodies (HAMA) using a validated sandwich ELISA kit (Biolegend). The threshold for HAMA-positive sera was set at >10 ng/mL. (B) ELISA-screened HAMA sera were incubated with anti-CD19-CAR-Jurkat T-cells. Jurkat wt cells served as CAR-negative staining controls. After incubation with PE-conjugated goat-anti human Fc detection antibody the cells were analyzed by flow cytometry. (C) HAMA-positive and -negative sera were selected and further tested for specific binding to anti-CD19 CAR-Jurkat T-cells by flow cytometry analysis. These sera were further used to assay for IgG effector mechanisms together with anti-CD19 CAR-Jurkat T-cells. (D) Sera from HLA-sensitized patients were screened for allogeneic reactivity towards anti-CD19 -CAR-Jurkat T-cells. Sera from OneLambda were used as HLA class I positive and negative control.

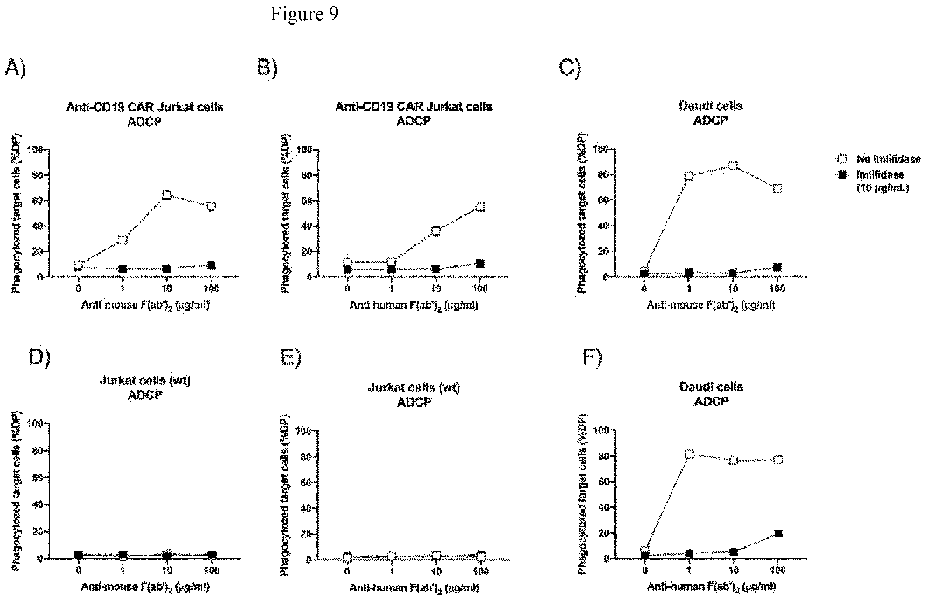

**Figure 9****: CAR-specific antibody-induced ADCP can be prevented by Imlifidase treatment - Polyclonal anti-F(ab')2 antibody opsonization of anti-CD19 CAR-Jurkat T-cells for ADCP is prevented by imlifidase treatment.** For flow cytometry based ADCP analysis the target cells, including (A, B) anti-CD19 CAR-Jurkat, (D, E) Jurkat wt cells, as well as (C, F) CD20 and BCR expressing Daudi cells, were stained with calcein-AM prior to incubation with imlifidase-treated rabbit (A, C, D) anti-mouse F(ab')2 or (B, E, F) anti-human F(ab')2 at indicated concentrations. The monocytic phagocytic effector cell line THP-1 was stained with CellTrace FarRed prior to being added to the target cells for 90 min. Phagocytosis was evaluated by flow cytometry. The amount of double positive cells reflecting phagocytized target cells are expressed as percentage of target cells.

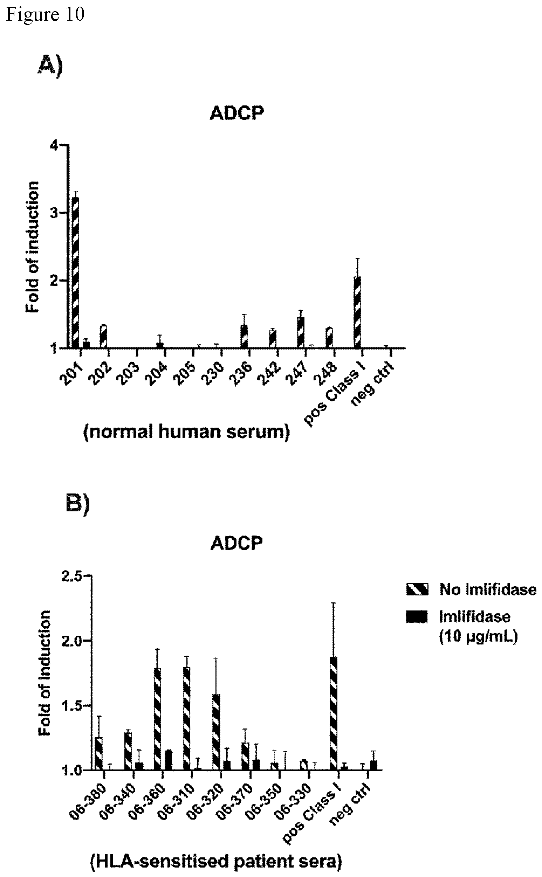

**Figure 10****: Imlifidase prevents ADCP-induction by allogeneic serum opsonized anti-CD19 CAR-Jurkat T-cells.** Target cells, anti-CD19 -CAR-Jurkat, were opsonized with sera from (A) healthy donors and (B) highly sensitized anti-HLA patients, with or without imlifidase (10 µg/mL) treatment. After washing, target cells were incubated for 6h at 37°C with FcγRI expressing reporter cells to allow induction of ADCP. The luciferase activity, a result of activated reporter cells, was measured using a luminescence reader, where luminescence signals (RLU) are presented as mean fold change of induction values ± SD.

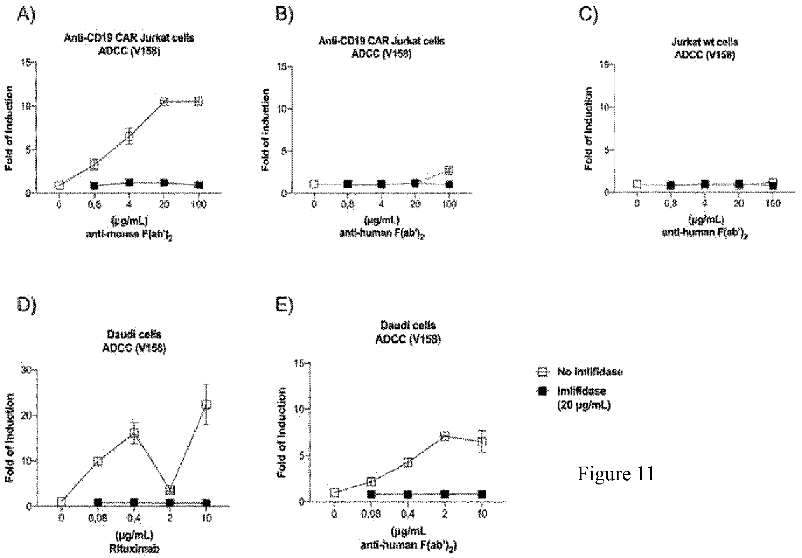

**Figure 11****: Anti-CD19 CAR-specific antibody-induced ADCC (V158) is prevented by imlifidase treatment.** Anti-CD19 CAR-Jurkat T-cells were opsonized with polyclonal rabbit (A) anti-mouse and (B) anti-human F(ab')₂-specific antibodies at indicated concentrations, with or without imlifidase (20 µg/mL). The induction of ADCC was quantified using a luciferase reporter bioassay of high affinity FcγRIIIa (V158) transfected reporter cells (Promega, #G7015). (C) CAR-negative Jurkat wt cells were treated with rabbit anti-human IgG, F(ab')₂ in presence/absence of imlifidase. BCR and CD20 positive Daudi cells, representing positive target cell controls for ADCC induction by (D) rituximab and (E) anti-human F(ab')₂-specific antibodies. The luminescence signals (RLU) derived from activated effector cells, are presented as mean fold change of induction values (of duplicates) ± SD.

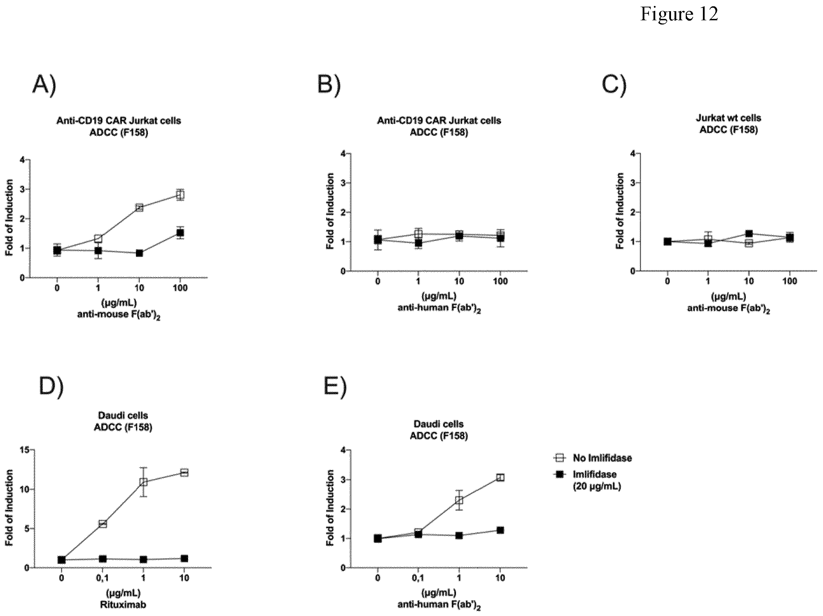

**Figure 12****: Anti-CD19 CAR-specific antibody-induced ADCC (F158) is prevented by imlifidase treatment.** Anti-CD19 CAR-Jurkat T-cells were opsonized with (A) polyclonal rabbit anti-mouse and (B) anti-human F(ab')₂-specific antibodies at indicated concentrations, with or without Imlifidase (20 µg/mL). The induction of ADCC was quantified using a luciferase reporter bioassay of low affinity FcγRIIIa (F158) transfected reporter cells (Promega, #G979A). (C) CAR-negative Jurkat wt cells were treated with rabbit anti-mouse IgG, F(ab')₂ in presence/absence of Imlifidase. BCR and CD20 positive Daudi cells, representing positive target cell controls for ADCC induction by (D) rituximab and (E) anti-human F(ab')₂-specific antibodies. The luminescence signals (RLU) derived from activated effector cells, are presented as mean fold change of induction values (of duplicates) ± SD.

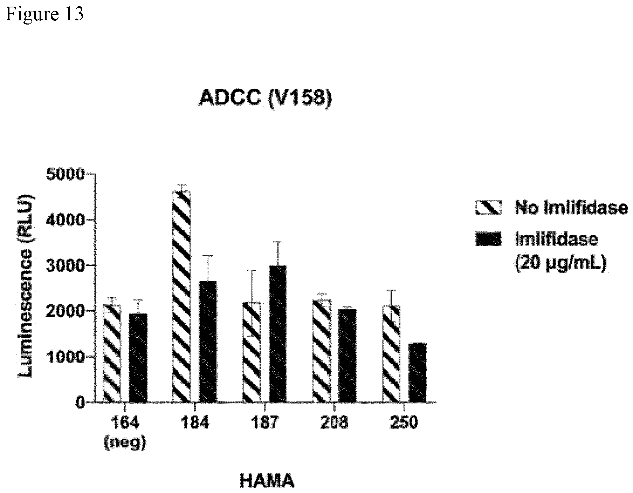

**Figure 13****: ADCC-induction by HAMA-opsonized anti-CD19 CAR-Jurkat T-cells can be prevented with imlifidase treatment.** Reporter cell lines expressing the CD16 FcγRIIIa high affinity (V158) allele were used to assay for ADCC induction. The murine mAb FMC63-based scFv-CD19-CAR Jurkat cell line was incubated with normal human serum samples, previously tested by ELISA for HAMA levels against murine IgG, in presence/absence of imlifidase HAMA-positive (184, 187, 208, 250) and HAMA-negative (164) human sera were included in the ADCC assay. The luminescence signals (RLU) derived from activated effector cells, are presented as mean values (of duplicates) ± SD.

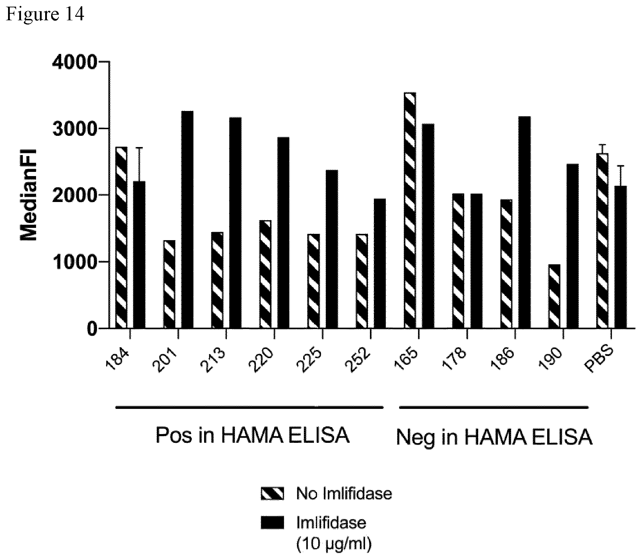

**Figure 14****: Imlifidase treatment of serum improves engagement of target CD19-protein with anti-CD19 CAR T-cells - CD19-protein binding to serum-exposed anti-CD19 CAR T-cells can be increased by imlifidase treatment.** Anti-CD19 CAR-Jurkat T-cells were incubated with HAMA-positive and -negative serum samples, with or without imlifidase (10 µg/mL). IHAc (1mM) was added to all samples to inactivate imlifidase during the next steps. Serum samples were incubated with anti-CD19 CAR-Jurkat T-cells to allow for possible IgG respectively F(ab')2 binding to the anti-CD19 CARs. After washing, recombinant atto-647N-labeled human CD19-Fc protein (ATM9269, R&D systems) was added to the cells and the anti-CD19 CAR and CD19-target protein interaction was evaluated by flow cytometry. Results are presented as median fluorescence intensity (FI).

### Brief Description of the Sequences

SEQ ID NO: 1 is the full sequence of IdeS including N terminal methionine and signal sequence. It is also available as NCBI Reference sequence no. WP_010922160.1
SEQ ID NO: 2 is the mature sequence of IdeS, lacking the N terminal methionine and signal sequence. It is also available as Genbank accession no. ADF13949.1
SEQ ID NO: 3 is the full sequence of IdeZ including N terminal methionine and signal sequence. It is also available as NCBI Reference sequence no. WP_014622780.1.
SEQ ID NO: 4 is the mature sequence of IdeZ, lacking the N terminal methionine and signal sequence.
SEQ ID NO: 5 is the sequence of a hybrid IdeS/Z. The N terminus is based on IdeZ lacking the N terminal methionine and signal sequence.
SEQ ID NOs: 6 to 25 are the sequences of exemplary proteases for use in the methods of the invention.
SEQ ID NO: 26 is the sequence of an IdeS polypeptide. Comprises the sequence of SEQ ID NO: 2 with an additional N terminal methionine and a histidine tag (internal reference pCART124).
SEQ ID NO: 27 is the sequence of an IdeZ polypeptide. Comprises the sequence of SEQ ID NO: 4 with an additional N terminal methionine and a histidine tag (internal reference pCART144).
SEQ ID NO: 28 is the sequence of an IdeS/Z polypeptide. Comprises the sequence of SEQ ID NO: 5 with an additional N terminal methionine and a histidine tag (internal reference pCART145).
SEQ ID NO: 29 is the contiguous sequence PLTPEQFRYNN, which corresponds to positions 63-73 of SEQ ID NO: 3.
SEQ ID NO: 30 is the contiguous sequence PPANFTQG, which corresponds to positions 58-65 of SEQ ID NO: 1.
SEQ ID NO: 31 is the contiguous sequence DDYQRNATEAYAKEVPHQIT, which corresponds to positions 35-54 of SEQ ID NO: 3.
SEQ ID NO: 32 is the contiguous sequence DSFSANQEIRYSEVTPYHVT, which corresponds to positions 30-49 of SEQ ID NO: 1.
SEQ ID NOs: 33 to 55 are nucleotide sequences encoding proteases set out above.
SEQ ID NOs: 56 to 69 are the sequences of exemplary proteases for use in the methods of the invention.
SEQ ID NO: 70 is the contiguous sequence NQTN, which corresponds to positions 336-339 of SEQ ID NO: 1.
SEQ ID NO: 71 is the contiguous sequence DSFSANQEIR YSEVTPYHVT, which corresponds to positions 30-49 of SEQ ID NO: 1.
SEQ ID NOs: 72 to 86 are nucleotide sequences encoding polypeptides disclosed herein.
SEQ ID NO: 87 is the sequence SFSANQEIRY SEVTPYHVT, which corresponds to positions 31-49 of SEQ ID NO: 1.
SEQ ID NO: 88 is the sequence DYQRNATEAY AKEVPHQIT, which corresponds to positions 36-54 of the IdeZ polypeptide NCBI Reference Sequence no WP_014622780.1.
SEQ ID NO: 89 is the sequence DDYQRNATEA YAKEVPHQIT, which may be present at the N terminus of a polypeptide of the invention.
SEQ ID NO: 90 shows the amino acid sequence of mature Endoglycosidase S (EndoS). Full sequence including secretion signal is available at Genbank Accession no. AAK00850.1.

### Detailed Description of the Invention

### Methods of improving the benefit to a patient of an adoptive cell transfer immunotherapy

The invention provides a protein that has IgG cysteine protease or IgG endoglycosidase activity for use in medicine, wherein the protein that has IgG cysteine protease or IgG endoglycosidase activity is used in combination with an adoptive cell transfer immunotherapy. The inventors have identified that the efficacy of adoptive cell transfer immunotherapies may be reduced by the limited survival and limited sustained activity of the transferred cells, such as CAR-T cells, and the inventors have shown in the examples that proteins with IgG cysteine protease or IgG endoglycosidase activity may protect transferred cells. In particular, the inventors have identified that cell surface receptor-specific antibodies may cut short the potential of transferred cells and the therapeutic effect of the transferred cells will profit from the removal of antibody effector functions through the conditioning of the recipient. Therefore, administering proteins with IgG cysteine protease or IgG endoglycosidase activity may increase the survival and activity of transferred cells and provide improved treatments. The inventors have also demonstrated that cell surface receptor-specific antibodies may interfere with the binding of an adoptive cell transfer immunotherapy receptor to its target. Therefore, administering proteins with IgG cysteine protease or IgG endoglycosidase activity may increase the potency and effect of an adoptive cell transfer immunotherapy.

The disclosure provides a method of prolonging the survival and/or enhancing the proliferation of cells administered as part of an adoptive cell transfer, comprising administering a protein that has IgG cysteine protease or IgG endoglycosidase activity prior to, subsequent to or concurrently with the adoptive cell transfer immunotherapy. The method may comprise administering a protein that has IgG cysteine protease or IgG endoglycosidase activity subsequent to a first adoptive cell transfer immunotherapy and prior to a second adoptive cell transfer immunotherapy.

The disclosure provides a method for conditioning or preparing a patient for an adoptive cell transfer immunotherapy, comprising administering a protein that has IgG cysteine protease or IgG endoglycosidase activity.

The disclosure provides a method for reducing serum IgG levels or reducing complement or Fc receptor binding by serum IgG molecules in a patient undergoing or scheduled to undergo an adoptive cell transfer immunotherapy.

The disclosure provides a method for increasing the potency of an adoptive cell transfer therapy, comprising administering a protein that has IgG cysteine protease or IgG endoglycosidase activity prior to, subsequent to or concurrently with the adoptive cell transfer immunotherapy. The disclosure provides a method for increasing the binding between the cell surface receptor of an adoptive cell transfer therapy and its target, comprising administering a protein that has IgG cysteine protease or IgG endoglycosidase activity prior to, subsequent to or concurrently with the adoptive cell transfer immunotherapy.

In certain embodiments, the improving the benefit to a patient of an adoptive cell transfer immunotherapy comprises administering a protein that has IgG cysteine protease or IgG endoglycosidase activity and subsequently administering an adoptive cell transfer immunotherapy. Such methods will allow pre-existing anti-drug antibodies (ADA) to be inactivated with the protein prior to administration of the cells, which will allow for better expansion and survival of the cells. ADA may bind any part of any cell therapy, including the expressed CAR or TCR or HLA antigens (in particular in allogenic therapies). The examples demonstrate that IdeS and EndoS are effective for inactivating pre-existing antibodies, for example IdeS and Endos were effective when used before addition of a complement source or effector cells. The examples also demonstrate that antibodies present in the serum of healthy individuals and HLA-sensitized patients that have not been administered an adoptive cell transfer immunotherapy are able to bind receptor constructs and cells such as CAR T-cells and mediate deleterious effects such as ADCP, ADCC and reduced target binding, all of which can be reduced by treatment with the proteins of the invention. The disclosure also provides a protein that has IgG cysteine protease or IgG endoglycosidase activity for use in conditioning a patient treatment by adoptive cell transfer immunotherapy. The invention also provides a protein that has IgG cysteine protease or IgG endoglycosidase activity for use in reducing serum IgG levels in a patient scheduled to receive an adoptive cell transfer immunotherapy treatment. The disclosure also provides a protein that has IgG cysteine protease or IgG endoglycosidase activity for use reducing in serum IgG levels in a patient that previously provided blood for development of an adoptive cell transfer immunotherapy and that has not yet received an adoptive cell transfer immunotherapy. The disclosure also provides an adoptive cell transfer immunotherapy composition for treating a patient that previously received administration of a protein that has IgG cysteine protease or IgG endoglycosidase activity.

In preferred embodiments, the improving the benefit to a patient of an adoptive cell transfer immunotherapy comprises administering a protein that has IgG cysteine protease or IgG endoglycosidase activity after administering the adoptive cell transfer immunotherapy. Such methods will allow pre-existing anti-drug antibodies (ADA) and antibodies elicited by the adoptive cell transfer immunotherapy to be inactivated. Expansion and survival of the transferred cells will therefore be improved. The examples demonstrate that IdeS and EndoS are effective for inactivating deleterious polyclonal antibodies and induced antibodies, because immune thrombocytopenia was reduced when Ides and EndoS were administered after the anti-platelet specific antibodies. The invention also provides a protein that has IgG cysteine protease or IgG endoglycosidase activity for use in improving the benefit to a patient of an adoptive cell transfer immunotherapy administered previously. The invention also provides a protein that has IgG cysteine protease or IgG endoglycosidase activity for use in treating a patient that previously received an adoptive cell transfer immunotherapy. The invention also provides a protein that has IgG cysteine protease or IgG endoglycosidase activity for use in reducing serum IgG levels in a patient that previously received an adoptive cell transfer immunotherapy treatment. The invention also provides an adoptive cell transfer immunotherapy composition for use in treating a patient that is scheduled to receive administration of a protein that has IgG cysteine protease or IgG endoglycosidase activity.

In further preferred embodiments, the improving the benefit to a patient of an adoptive cell transfer immunotherapy comprises administering a protein that has IgG cysteine protease or IgG endoglycosidase activity both before and after administering doses of an adoptive cell transfer immunotherapy. Such methods will allow both pre-existing anti-drug antibodies (ADA) and antibodies elicited by the adoptive cell transfer immunotherapy to be inactivated. Expansion and survival of the transferred cells will therefore be improved.

In further preferred embodiments, the improving the benefit to a patient of an adoptive cell transfer immunotherapy comprises administering a protein that has IgG cysteine protease or IgG endoglycosidase activity after administering a first adoptive cell transfer immunotherapy and prior to administering a second adoptive cell transfer immunotherapy. Thus the protein is administered between two or more doses of adoptive cell transfer immunotherapy. Such methods will allow for better expansion and survival of transferred cells because any ADA from previous injections will be inactivated. Preferably in such embodiments, the first and second, and any subsequent, adoptive cell transfer immunotherapies use the same or similar constructs and cells. In such embodiments, similar constructs or cells may have ADA cross reactive epitopes. The invention also provides a protein that has IgG cysteine protease or IgG endoglycosidase activity for use in treating a patient that previously received a first dose of an adoptive cell transfer immunotherapy and that is scheduled to receive a second dose of an adoptive cell transfer immunotherapy. The invention also provides a protein that has IgG cysteine protease or IgG endoglycosidase activity for use in reducing IgG levels in a patient that is undergoing a multiple dose regime of an adoptive cell transfer immunotherapy. The invention also provides a protein that has IgG cysteine protease or IgG endoglycosidase activity for use in improving the benefit to a patient of a multiple dose regime adoptive cell transfer immunotherapy treatment. The invention also provides an adoptive cell transfer immunotherapy composition for treating a patient that previously has received a dose of an adoptive cell transfer immunotherapy composition and previously received administration of a protein that has IgG cysteine protease or IgG endoglycosidase activity.

In further preferred embodiments, the method of improving the benefit to a patient of an adoptive cell transfer immunotherapy comprises administering a protein that has IgG cysteine protease or IgG endoglycosidase activity after administering two or more doses of an adoptive cell transfer immunotherapy. Such methods will allow antibodies elicited by the adoptive cell transfer immunotherapy to be inactivated. Expansion and survival of the transferred cells will therefore be improved. The invention also provides a protein that has IgG cysteine protease or IgG endoglycosidase activity for use in improving the benefit to a patient of an adoptive cell transfer immunotherapy administered previously in two or more doses. The invention also provides a protein that has IgG cysteine protease or IgG endoglycosidase activity for use in treating a patient that previously received two or more doses of an adoptive cell transfer immunotherapy. The invention also provides a protein that has IgG cysteine protease or IgG endoglycosidase activity for use in reducing serum IgG levels in a patient that previously received two or more doses of an adoptive cell transfer immunotherapy treatment.

In certain embodiments of the invention, the improving the benefit to a patient of an adoptive cell transfer immunotherapy comprises multiple administrations of a protein that has IgG cysteine protease or IgG endoglycosidase activity and multiple administrations of the adoptive cell transfer immunotherapy. In certain embodiments, the multiple administrations of a protein that has IgG cysteine protease or IgG endoglycosidase activity comprises administration of the same enzyme. Repeat doses of a protein that has IgG cysteine protease or IgG endoglycosidase activity may be separated by any appropriate time period, such as 1-7 days, 5-7 days or 6-8 days. In certain embodiments, the multiple administrations of a protein that has IgG cysteine protease or IgG endoglycosidase activity comprises administration of different enzymes.

In certain embodiments of the invention, the improving the benefit to a patient of an adoptive cell transfer immunotherapy comprises concurrent administration of a protein that has IgG cysteine protease or IgG endoglycosidase activity and the adoptive cell transfer immunotherapy. The examples demonstrate that cell surface receptor-specific antibodies may interfere with the binding of an adoptive cell transfer immunotherapy receptor to its target, but binding can be increased with treatment of a protein of the invention, so concurrent administration of proteins with IgG cysteine protease or IgG endoglycosidase activity may increase the potency and effect of an adoptive cell transfer immunotherapy.

In preferred embodiments of the method of the invention, administration of a protein that has IgG cysteine protease or IgG endoglycosidase activity inactivates all or substantially all IgG molecules present in the serum of the patient. In certain embodiments, the protein is administered in an amount sufficient to eliminate Fc receptor or complement binding by all or substantially all IgG molecules present in the serum of the patient.

In uses of the invention wherein the protein that has IgG cysteine protease or IgG endoglycosidase activity is administered prior to a dose of adoptive cell transfer immunotherapy, the two administrations are separated by a time interval which is preferably sufficient for inactivation or elimination of Fc receptor and complement binding for all or substantially all IgG molecules present in the serum of the subject. The said interval may typically be of at least 30 minutes and typically at most 21 days. Administration of the protein that has IgG cysteine protease or IgG endoglycosidase activity may occur concurrently with lymphodepletion (such as on the same day), or 1-7 days prior or subsequent to lymphodepletion. In certain embodiments, the administration of the protein occurs between lymphodepletion and administration of the cell therapy. By "substantially all" it is typically meant that Fc receptor and complement binding by serum IgG is reduced to less than 30%, 20%, 15%, 10% or 5% of the level that was present prior to administration. For example, if the protein is a protease (such as IdeS), the interval will be the time required for the agent to cleave at least 70%, 80%, 85%, 90% or 95% of serum IgG in the subject, as measured by any suitable assay in the subject, as measured by any suitable assay.

In uses of the invention wherein the protein that has IgG cysteine protease or IgG endoglycosidase activity is administered subsequent to a dose of adoptive cell transfer immunotherapy, the protein is preferably administered during proliferation of the transferred cells, such as within 2 weeks, 1 week, 2 days, 1 day or 5 hours of the administration of the adoptive cell transfer immunotherapy.

In preferred embodiments, the uses of the invention are for reducing antibody-mediated complement deposition, CDC, ADCC, ADCP, exhaustion or receptor activated cell death of cells previously administered to a patient in an adoptive cell transfer immunotherapy. In other preferred embodiments, the uses of the invention are for reducing antibody-mediated complement deposition, CDC, ADCC, ADCP, exhaustion or receptor activated cell death of cells to be subsequently administered to a patient in an adoptive cell transfer immunotherapy. In preferred embodiments, the invention provides a protein that has IgG cysteine protease or IgG endoglycosidase activity for use in reducing antibody-mediated complement deposition, CDC, ADCC, ADCP, exhaustion or receptor activated cell death of cells previously administered to a patient in an adoptive cell transfer immunotherapy. In other preferred embodiments, the invention provides a protein that has IgG cysteine protease or IgG endoglycosidase activity for use in reducing antibody-mediated complement deposition, CDC, ADCC, ADCP, exhaustion or receptor activated cell death to be subsequently administered to a patient in an adoptive cell transfer immunotherapy. In preferred embodiments, the ADCC is mediated by FcγRIIIa (V158) effector cells or FcγRIIIa (F158) effector cells.

In certain embodiments, the method of the invention comprises administering a protein that has IgG cysteine protease or IgG endoglycosidase activity to a patient that has detectable levels of human anti-mouse antibodies (HAMA), which are immunoglobulins with specificity for mouse immunoglobulins. Said administering may be prior to, subsequent to, or concurrent with administration of an adoptive cell transfer immunotherapy. The examples demonstrate that HAMA in patient sera can exert deleterious effects on adoptive cell transfer immunotherapy cells but these effects can be reduced or prevented by treatment with a protein of the invention. HAMA may be induced in normal individuals from contact with murine antigens. The frequency and concentration of HAMA can be expected to be even higher in patients receiving murine mAb-based biologics, in some cases even leading to partial neutralization of these therapeutics.

In the uses of the invention, the IgG cysteine protease or IgG endoglycosidase may be co-administered with an immune-suppressive agent. In the uses of the invention, the protease is preferably administered by intravenous infusion, but may be administered by any suitable route including, for example, intradermal, subcutaneous, percutaneous, intramuscular, intra-arterial, intraperitoneal, intraarticular, intraosseous, intrathecal, intraventricular or other appropriate administration routes. The amount of the protease or endoglycosidase that is administered may be between 0.01 mg/kg BW and 2 mg/kg BW, between 0.05 and 1.5 mg/kg BW, between 0.1 mg/kg BW and 1 mg/kg BW, preferably between 0.15 mg/kg and 0.7 mg/kg BW and most preferably between 0.2 mg/kg and 0.3 mg/kg BW, in particular 0.25 mg/kg BW. The protein may be administered on multiple occasions to the same subject, provided that the quantity of anti-drug antibody (ADA) in the serum of the subject which is capable of binding to the protein does not exceed a threshold determined by the clinician. The quantity of ADA in the serum of the subject which is capable of binding to the protease may be determined by any suitable method, such as an agent specific CAP FEIA (ImmunoCAP) test or a titre assay.

### Treating cancer

The invention provides a protein that has IgG cysteine protease or IgG endoglycosidase activity for use in a method for treating cancer, wherein the protein that has IgG cysteine protease or IgG endoglycosidase activity is used in combination with an adoptive cell transfer immunotherapy. The protection of cells shown in the examples indicates that administering proteins with IgG cysteine protease or IgG endoglycosidase activity may increase the survival and activity of transferred cells and provide improved cancer treatment.

In certain embodiments, the treating cancer comprises administering a protein that has IgG cysteine protease or IgG endoglycosidase activity and subsequently administering an adoptive cell transfer immunotherapy. The invention also provides a protein that has IgG cysteine protease or IgG endoglycosidase activity for use in conditioning a patient for cancer treatment by adoptive cell transfer immunotherapy. The invention also provides a protein that has IgG cysteine protease or IgG endoglycosidase activity for use in reducing serum IgG levels in a patient scheduled to receive an adoptive cell transfer immunotherapy cancer treatment. The invention also provides a protein that has IgG cysteine protease or IgG endoglycosidase activity for use reducing in serum IgG levels in a patient that previously provided blood for development of an adoptive cell transfer immunotherapy and that has not yet received an adoptive cell transfer immunotherapy. The invention also provides an adoptive cell transfer immunotherapy composition for use in treating cancer in a patient that previously received administration of a protein that has IgG cysteine protease or IgG endoglycosidase activity.

In preferred embodiments, the treating cancer comprises administering a protein that has IgG cysteine protease or IgG endoglycosidase activity after administering an adoptive cell transfer immunotherapy. The invention also provides a protein that has IgG cysteine protease or IgG endoglycosidase activity for use in improving the benefit to a patient of an adoptive cell transfer immunotherapy administered previously. The invention also provides a protein that has IgG cysteine protease or IgG endoglycosidase activity for use in treating cancer in a patient that previously received an adoptive cell transfer immunotherapy. The invention also provides a protein that has IgG cysteine protease or IgG endoglycosidase activity for use in reducing serum IgG levels in a patient that previously received an adoptive cell transfer immunotherapy cancer treatment. The invention also provides an adoptive cell transfer immunotherapy composition for treating cancer in a patient that is scheduled to receive administration of a protein that has IgG cysteine protease or IgG endoglycosidase activity.

In further preferred embodiments, treating cancer comprises administering a protein that has IgG cysteine protease or IgG endoglycosidase activity both before and after administering doses of an adoptive cell transfer immunotherapy.

In further preferred embodiments, treating cancer comprises administering a protein that has IgG cysteine protease or IgG endoglycosidase activity after administering a first adoptive cell transfer immunotherapy and prior to administering a second adoptive cell transfer immunotherapy. Preferably in such embodiments, the first and second, and any subsequent, adoptive cell transfer immunotherapies use the same or similar constructs and cells. The invention also provides a protein that has IgG cysteine protease or IgG endoglycosidase activity for use in treating cancer in a patient that previously received a first dose of an adoptive cell transfer immunotherapy and that is scheduled to receive a second dose of an adoptive cell transfer immunotherapy. The invention also provides a protein that has IgG cysteine protease or IgG endoglycosidase activity for use in reducing IgG levels in a patient that is undergoing a multiple dose regime of an adoptive cell transfer immunotherapy. The invention also provides a protein that has IgG cysteine protease or IgG endoglycosidase activity for use in improving the benefit to a patient of a multiple dose regime adoptive cell transfer immunotherapy cancer treatment. The invention also provides an adoptive cell transfer immunotherapy composition for use in treating cancer in a patient that previously has received a dose of an adoptive cell transfer immunotherapy composition and previously received administration of a protein that has IgG cysteine protease or IgG endoglycosidase activity.

In further preferred embodiments, treating cancer comprises administering a protein that has IgG cysteine protease or IgG endoglycosidase activity after administering two or more doses of an adoptive cell transfer immunotherapy. The invention also provides a protein that has IgG cysteine protease or IgG endoglycosidase activity for use in improving the benefit to a patient of an adoptive cell transfer immunotherapy administered previously in two or more doses. The invention also provides a protein that has IgG cysteine protease or IgG endoglycosidase activity for use in treating cancer in a patient that previously received two or more doses of an adoptive cell transfer immunotherapy. The invention also provides a protein that has IgG cysteine protease or IgG endoglycosidase activity for use in reducing serum IgG levels in a patient that previously received two or more doses of an adoptive cell transfer immunotherapy cancer treatment.

In certain embodiments of the invention, treating cancer comprises multiple administrations of a protein that has IgG cysteine protease or IgG endoglycosidase activity and multiple administrations of an adoptive cell transfer immunotherapy.

In certain embodiments of the invention, treating cancer comprises concurrent administration of a protein that has IgG cysteine protease or IgG endoglycosidase activity and an adoptive cell transfer immunotherapy.

In the therapeutic uses of the invention, proteins and adoptive cell transfer immunotherapies are administered to a subject already suffering from cancer, in an amount sufficient to cure, alleviate or partially arrest the cancer or one or more of its symptoms. Such therapeutic treatment may result in remission, stabilisation, reduction in metastasis or elimination of the cancer. An amount adequate to accomplish this is defined as "therapeutically effective amount". The subject may have been identified as suffering from cancer and being suitable for an adoptive cell transfer immunotherapy by any suitable means.

### Treating autoimmune conditions

The invention provides a protein that has IgG cysteine protease or IgG endoglycosidase activity for use in treating autoimmune conditions, wherein protein that has IgG cysteine protease or IgG endoglycosidase activity is used in combination with an adoptive cell transfer immunotherapy. The protection of cells shown in the examples indicates that administering proteins with IgG cysteine protease or IgG endoglycosidase activity may increase the survival and activity of transferred cells and provide improved treatment of autoimmune conditions.

In preferred embodiments, treating autoimmune conditions comprises administering a protein that has IgG cysteine protease or IgG endoglycosidase activity and subsequently administering an adoptive cell transfer immunotherapy. The invention also provides a protein that has IgG cysteine protease or IgG endoglycosidase activity for use in conditioning a patient for treatment of an autoimmune condition by adoptive cell transfer immunotherapy. The invention also provides a protein that has IgG cysteine protease or IgG endoglycosidase activity for use in reducing serum IgG levels in a patient scheduled to receive an adoptive cell transfer immunotherapy treatment for an autoimmune condition. The invention also provides a protein that has IgG cysteine protease or IgG endoglycosidase activity for use reducing in serum IgG levels in a patient that previously provided blood for development of an adoptive cell transfer immunotherapy and that has not yet received an adoptive cell transfer immunotherapy. The invention also provides an adoptive cell transfer immunotherapy composition for treating an autoimmune condition in a patient that previously received administration of a protein that has IgG cysteine protease or IgG endoglycosidase activity.

In further preferred embodiments, treating an autoimmune condition comprises administering a protein that has IgG cysteine protease or IgG endoglycosidase activity after administering an adoptive cell transfer immunotherapy. The invention also provides a protein that has IgG cysteine protease or IgG endoglycosidase activity for use in improving the benefit to a patient of an adoptive cell transfer immunotherapy administered previously. The invention also provides a protein that has IgG cysteine protease or IgG endoglycosidase activity for use in treating an autoimmune condition in a patient that previously received an adoptive cell transfer immunotherapy. The invention also provides a protein that has IgG cysteine protease or IgG endoglycosidase activity for use in reducing serum IgG levels in a patient that previously received an adoptive cell transfer immunotherapy treatment for an autoimmune condition. The invention also provides an adoptive cell transfer immunotherapy composition for use in treating an autoimmune condition in a patient that is scheduled to receive administration of a protein that has IgG cysteine protease or IgG endoglycosidase activity.

In further preferred embodiments, treating an autoimmune condition comprises administering a protein that has IgG cysteine protease or IgG endoglycosidase activity both before and after administering doses of an adoptive cell transfer immunotherapy.

In further preferred embodiments, treating an autoimmune condition comprises administering a protein that has IgG cysteine protease or IgG endoglycosidase activity after administering a first adoptive cell transfer immunotherapy and prior to administering a second adoptive cell transfer immunotherapy. Preferably in such embodiments, the first and second, and any subsequent, adoptive cell transfer immunotherapies use the same or similar constructs and cells. The invention also provides a protein that has IgG cysteine protease or IgG endoglycosidase activity for use in treating an autoimmune condition in a patient that previously received a first dose of an adoptive cell transfer immunotherapy and that is scheduled to receive a second dose of an adoptive cell transfer immunotherapy. The invention also provides a protein that has IgG cysteine protease or IgG endoglycosidase activity for use in reducing IgG levels in a patient that is undergoing a multiple dose regime of an adoptive cell transfer immunotherapy. The invention also provides a protein that has IgG cysteine protease or IgG endoglycosidase activity for use in improving the benefit to a patient of a multiple dose regime adoptive cell transfer immunotherapy treatment for an autoimmune condition. The invention also provides an adoptive cell transfer immunotherapy composition for treating an autoimmune condition in a patient that previously has received a dose of an adoptive cell transfer immunotherapy composition and previously received administration of a protein that has IgG cysteine protease or IgG endoglycosidase activity.

In further preferred embodiments, treating an autoimmune condition comprises administering a protein that has IgG cysteine protease or IgG endoglycosidase activity after administering two or more doses of an adoptive cell transfer immunotherapy. The invention also provides a protein that has IgG cysteine protease or IgG endoglycosidase activity for use in improving the benefit to a patient of an adoptive cell transfer immunotherapy administered previously in two or more doses. The invention also provides a protein that has IgG cysteine protease or IgG endoglycosidase activity for use in treating an autoimmune condition in a patient that previously received two or more doses of an adoptive cell transfer immunotherapy. The invention also provides a protein that has IgG cysteine protease or IgG endoglycosidase activity for use in reducing serum IgG levels in a patient that previously received two or more doses of an adoptive cell transfer immunotherapy treatment for an autoimmune condition.

In certain embodiments of the invention, treating an autoimmune condition comprises multiple administrations of a protein that has IgG cysteine protease or IgG endoglycosidase activity and multiple administrations of an adoptive cell transfer immunotherapy.

In the therapeutic uses of the invention, proteins and adoptive cell transfer immunotherapies are administered to a subject already suffering from an autoimmune condition, in an amount sufficient to cure, alleviate or partially arrest the autoimmune condition or one or more of its symptoms. Such therapeutic use may result in remission, stabilisation, reduction in flare ups or elimination of the autoimmune condition. An amount adequate to accomplish this is defined as "therapeutically effective amount". The subject may have been identified as suffering from an autoimmune condition and being suitable for an adoptive cell transfer immunotherapy by any suitable means.

### Treating infections

The invention provides a protein that has IgG cysteine protease or IgG endoglycosidase activity for use in treating an infection wherein the protein that has IgG cysteine protease or IgG endoglycosidase activity is used in combination with an adoptive cell transfer immunotherapy. The protection of cells shown in the examples indicates that administering proteins with IgG cysteine protease or IgG endoglycosidase activity may increase the survival and activity of transferred cells and provide improved infection treatment.

In certain embodiments, treating an infection comprises administering a protein that has IgG cysteine protease or IgG endoglycosidase activity and subsequently administering an adoptive cell transfer immunotherapy. The invention also provides a protein that has IgG cysteine protease or IgG endoglycosidase activity for use in conditioning a patient for infection treatment by adoptive cell transfer immunotherapy. The invention also provides a protein that has IgG cysteine protease or IgG endoglycosidase activity for use in reducing serum IgG levels in a patient scheduled to receive an adoptive cell transfer immunotherapy infection treatment. The invention also provides a protein that has IgG cysteine protease or IgG endoglycosidase activity for use reducing in serum IgG levels in a patient that previously provided blood for development of an adoptive cell transfer immunotherapy and that has not yet received an adoptive cell transfer immunotherapy. The invention also provides an adoptive cell transfer immunotherapy composition for treating an infection in a patient that previously received administration of a protein that has IgG cysteine protease or IgG endoglycosidase activity.

In preferred embodiments, treating an infection comprises administering a protein that has IgG cysteine protease or IgG endoglycosidase activity after administering an adoptive cell transfer immunotherapy. The invention also provides a protein that has IgG cysteine protease or IgG endoglycosidase activity for use in improving the benefit to a patient of an adoptive cell transfer immunotherapy administered previously. The invention also provides a protein that has IgG cysteine protease or IgG endoglycosidase activity for use in treating an infection in a patient that previously received an adoptive cell transfer immunotherapy. The invention also provides a protein that has IgG cysteine protease or IgG endoglycosidase activity for use in reducing serum IgG levels in a patient that previously received an adoptive cell transfer immunotherapy infection treatment. The invention also provides an adoptive cell transfer immunotherapy composition for treating an infection in a patient that is scheduled to receive administration of a protein that has IgG cysteine protease or IgG endoglycosidase activity.

In further preferred embodiments, treating an infection comprises administering a protein that has IgG cysteine protease or IgG endoglycosidase activity both before and after administering doses of an adoptive cell transfer immunotherapy.

In further preferred embodiments, treating an infection comprises administering a protein that has IgG cysteine protease or IgG endoglycosidase activity after administering a first adoptive cell transfer immunotherapy and prior to administering a second adoptive cell transfer immunotherapy. Preferably in such embodiments, the first and second, and any subsequent, adoptive cell transfer immunotherapies use the same or similar constructs and cells. The invention also provides a protein that has IgG cysteine protease or IgG endoglycosidase activity for use in treating an infection in a patient that previously received a first dose of an adoptive cell transfer immunotherapy and that is scheduled to receive a second dose of an adoptive cell transfer immunotherapy. The invention also provides a protein that has IgG cysteine protease or IgG endoglycosidase activity for use in reducing IgG levels in a patient that is undergoing a multiple dose regime of an adoptive cell transfer immunotherapy. The invention also provides a protein that has IgG cysteine protease or IgG endoglycosidase activity for use in improving the benefit to a patient of a multiple dose regime adoptive cell transfer immunotherapy infection treatment. The invention also provides an adoptive cell transfer immunotherapy composition for use in treating an infection in a patient that previously has received a dose of an adoptive cell transfer immunotherapy composition and previously received administration of a protein that has IgG cysteine protease or IgG endoglycosidase activity.

In further preferred embodiments, treating an infection comprises administering a protein that has IgG cysteine protease or IgG endoglycosidase activity after administering two or more doses of an adoptive cell transfer immunotherapy. The invention also provides a protein that has IgG cysteine protease or IgG endoglycosidase activity for use in improving the benefit to a patient of an adoptive cell transfer immunotherapy administered previously in two or more doses. The invention also provides a protein that has IgG cysteine protease or IgG endoglycosidase activity for use in treating an infection in a patient that previously received two or more doses of an adoptive cell transfer immunotherapy. The invention also provides a protein that has IgG cysteine protease or IgG endoglycosidase activity for use in reducing serum IgG levels in a patient that previously received two or more doses of an adoptive cell transfer immunotherapy infection treatment.

In certain embodiments of the invention, treating an infection comprises multiple administrations of a protein that has IgG cysteine protease or IgG endoglycosidase activity and multiple administrations of an adoptive cell transfer immunotherapy.

In certain embodiments of the invention, treating an infection comprises concurrent administration of a protein that has IgG cysteine protease or IgG endoglycosidase activity and an adoptive cell transfer immunotherapy.

In the therapeutic uses of the invention, proteins and adoptive cell transfer immunotherapies are administered to a subject diagnosed with an infection, in an amount sufficient to reduce or eliminate the virus from the patient. Such therapeutic treatment may result in a reduction in viral load or cure of the patient. An amount adequate to accomplish this is defined as "therapeutically effective amount". The subject may have been identified as suffering from an infection and being suitable for an adoptive cell transfer immunotherapy by any suitable means.

### Treating conditions mediated by B-cells producing deleterious antibodies

The invention provides also a protein that has IgG cysteine protease or IgG endoglycosidase activity for use in treating conditions mediated by B-cells producing deleterious antibodies, wherein the protein that has IgG cysteine protease or IgG endoglycosidase activity is used in combination with an adoptive cell transfer immunotherapy. The protection of cells shown in the examples indicates that administering proteins with IgG cysteine protease or IgG endoglycosidase activity may increase the survival and activity of transferred cells and provide improved treatment of conditions mediated by B-cells producing deleterious antibodies. In certain embodiments, the deleterious antibodies are pathogenic antibodies.

Examples of conditions mediated by B-cells producing deleterious antibodies include autoimmune conditions wherein B-cells producing antibodies against particular antigens mediate the disease process. Using an adoptive cell transfer immunotherapy may allow elimination of the problematic B-cells. Similarly, B-cells that produce anti-drug-antibodies against a key therapy may be a problem that can be eliminated by an adoptive cell transfer immunotherapy. In these cases, the adoptive cell transfer immunotherapy may express the antigen that is targeted by the problematic B-cell.

In certain embodiments, treating the condition comprises administering a protein that has IgG cysteine protease or IgG endoglycosidase activity and subsequently administering an adoptive cell transfer immunotherapy.

In preferred embodiments, treating the condition comprises administering a protein that has IgG cysteine protease or IgG endoglycosidase activity after administering an adoptive cell transfer immunotherapy.

In further preferred embodiments, treating the condition comprises administering a protein that has IgG cysteine protease or IgG endoglycosidase activity both before and after administering doses of an adoptive cell transfer immunotherapy.

In further preferred embodiments, treating the condition comprises administering a protein that has IgG cysteine protease or IgG endoglycosidase activity after administering a first adoptive cell transfer immunotherapy and prior to administering a second adoptive cell transfer immunotherapy. Preferably in such embodiments, the first and second, and any subsequent, adoptive cell transfer immunotherapies use the same or similar constructs and cells.

In further preferred embodiments, treating the condition comprises administering a protein that has IgG cysteine protease or IgG endoglycosidase activity after administering two or more doses of an adoptive cell transfer immunotherapy.

In certain embodiments of the invention, treating the condition comprises multiple administrations of a protein that has IgG cysteine protease or IgG endoglycosidase activity and multiple administrations of an adoptive cell transfer immunotherapy.

In certain embodiments of the invention, treating the condition comprises concurrent administration of a protein that has IgG cysteine protease or IgG endoglycosidase activity and an adoptive cell transfer immunotherapy.

In the therapeutic uses of the invention, proteins and adoptive cell transfer immunotherapies are administered to a subject already suffering from condition mediated by B-cell producing deleterious antibodies, in an amount sufficient to cure, alleviate or partially arrest the autoimmune condition or one or more of its symptoms. Such therapeutic treatment may result in remission, stabilisation, reduction in flare ups or elimination of the condition. An amount adequate to accomplish this is defined as "therapeutically effective amount". The subject may have been identified as suffering from the condition and being suitable for an adoptive cell transfer immunotherapy by any suitable means.

### Adoptive cell transfer immunotherapies

The uses of the invention provide increased benefits from adoptive cell transfer (ACT) immunotherapies and thereby provide improved methods of treating cancer, autoimmune conditions, infections and conditions mediated by B-cells producing deleterious antibodies. ACT immunotherapies are an established and potent approach for treating cancer in particular. ACT is the passive transfer of *ex vivo* grown cells, most commonly immune-derived cells, into a host with the goal of transferring the immunologic functionality and characteristics of the transplant.

ACT can be autologous (e.g., isolated by leukapheresis, transduced and selected approximately 4 weeks immediately prior to administration), as is common in adoptive T-cell therapies, or allogeneic, in which case the methods of the invention may improve the ACT by removing antibodies that recognise the expressed receptor and/or other antigens on the allogenic cells. Moreover, the ACT may be xenogeneic. In preferred embodiments, ACT is autologous.

ACT may also comprise transfer of autologous tumor infiltrating lymphocytes (TILs) which may be used to treat patients with advanced solid tumors such as melanoma and hematologic malignancies.

ACT may also comprise transfer of allogeneic lymphocytes isolated, prepared, and stored (e.g., frozen) "off-the-shelf" from a healthy donor which may be used to treat patients with advanced solid tumors, such as melanoma, and hematologic malignancies.

The adoptive cell immunotherapy of the invention may include administration of cells expressing a chimeric antigen receptor (CAR), or a T-cell receptor (TCR), or may include tumor-infiltrating lymphocytes (TIL). The population of cells expressing the CAR/TCR, which recognize an antigen, may comprise a population of activated T-cells or natural killer (NK) cells or dendritic cells. Dendritic cells are capable of antigen presentation, as well as direct killing of tumors. Dendritic cells may express, for example, 4-1BB or an anti-CD19 CAR. The population of cells expressing the CAR/TCR may comprise a population of gene-edited cells.

The ACT may use cell types such as T-cells, natural killer (NK) cells, delta-gamma T-cells, regulatory T-cells, dendritic cells, and peripheral blood mononuclear cells. The ACT may use monocytes with the purpose of inducing differentiation to dendritic cells and/or macrophages subsequent to contact with tumor antigens.

According to preferred embodiments of the invention, the adoptive cell therapy may be a CAR T-cell therapy. The CAR T-cell can be engineered to target a tumour antigen of interest by way of engineering a desired antigen binding domain that specifically binds to an antigen on a tumour cell. In preferred embodiments, the cell therapy uses a cell of hematopoietic origin. The examples demonstrate that the methods of the invention are particularly effective against cells of hematopoietic origin.

In preferred embodiments, the CAR T-cell therapy employs CAR T-cells that target CD19, CD20, CD22, CD30, CD33, CD38, CD123, CD138, CS-1, B-cell maturation antigen (BCMA), MAGEA3, MAGEA3/A6, KRAS, CLL1, MUC-1, HER2, EpCam, GD2, GPA7, PSCA, EGFR, EGFRvIII, ROR1, mesothelin, CD33/IL3Ra, c-Met, CD37, PSMA, Glycolipid F77, GD-2, gp100, NY-ESO-1 TCR, FRalpha, CD24, CD44, CD133, CD166, CA-125, HE4, Oval, estrogen receptor, progesterone receptor, uPA, PAI-1, MICA, MICB, ULBP1, ULBP2, ULBP3, ULBP4, ULBP5 or ULBP6, or a combination thereof (e.g., both CD33 and CD123). Preferred antigens are BCMA, CD19, CD20 and CD22.

A preferred adoptive cell transfer immunotherapy is CAR T-cell therapy (e.g., autologous cell therapy and allogeneic cell therapy). Preferred are CAR T-cell therapies for treating hematologic malignancies such as ALL, AML, NHL, DLBCL and CLL. Examples of approved CAR T-cell therapies include, without limitation, KYMRIAH^{®} (tisagenlecleucel) for treating NHL and DLBCL, and YESCARTA^{®} (axicabtagene ciloleucel) for treating NHL.

According to certain aspects of the present invention, the population of cells expressing the CAR/TCR or the TIL may be autologous cells, allogeneic cells derived from another human donor, or xenogeneic cells derived from an animal of a different species.

According to certain aspects of the present invention, the population of cells expressing the CAR/TCR or the TIL may be isolated by leukapheresis, transduced and selected approximately 4 weeks immediately prior to administration, as in the case of autologous stem cells, or may be isolated from a healthy donor and prepared in advance then stored, such as a frozen preparation, for one or more patients as in the case of so called "off-the-shelf" allogeneic CAR-T stem cell therapies.

According to certain aspects of the present invention, the population of cells expressing the CAR/TCR may comprise a population of activated T-cells or natural killer (NK) cells or dendritic cells expressing the CAR/TCR which recognize an antigen. Dendritic cells are capable of antigen presentation, as well as direct killing of tumours.

According to certain aspects of the present invention, the antigen may be one that is expressed only on cancer cells or one that is preferentially expressed on cancer cells, such as a neo-antigen or lineage-specific antigen (such as CD19 or CD20).

The CAR T-cell may comprise an antigen binding domain capable of targeting two or more different antigens (i.e., bispecific or bivalent, trispecific or trivalent, tetraspecific, etc.). As such, the CAR T-cell may comprise a first antigen binding domain that binds to a first antigen and a second antigen binding domain that binds to a second antigen (e.g., tandem CAR). For example, the CAR T-cell may comprise a CD19 binding domain and a CD22 binding domain and may thus recognize and bind to both CD19 and CD22. Or further, the CAR T-cell may comprise a CD19 binding domain and a CD20 binding domain and may thus recognize and bind to both CD19 and CD20.

Alternatively, each cell in the population of cells, or the overall population of cells, may comprise more than one distinct CAR T-cell (e.g., construct), wherein each CAR T-cell construct may recognize a different antigen. For example, the population of CAR T-cells may target three antigens such as, for example, HER2, IL13Rα2, and EphA2.

According to certain aspects of the present invention, the population of cells, whether autologous or allogeneic, may be engineered using gene editing technology such as by CRISPR/cas9 (clustered regularly interspaced short palindromic repeats/ CRISPR associated protein 9), Zinc Finger Nucleases (ZFN), or transcription activator-like effector nuclease (TALEN). These technologies, recognized and practiced in the art of genetic engineering, enable selective editing, disruption, or insertion of targeted sequences to modify the genome of the cell of interest. Accordingly, isolated autologous or allogeneic cells for adoptive transfer practiced in the current invention may be edited to delete or replace a known gene or sequence. For example, the T cell receptor (TCR) in an allogeneic T cell population may be deleted or replaced prior to or after CAR-T transduction as a means to eliminate graft-versus-host disease in recipient patients.

According to certain aspects of the present invention, the population of cells administered as the adoptive cell transfer immunotherapy may comprise a population of T-cells, NK-cells, or dendritic cells expressing a CAR, wherein the CAR comprises an extracellular antibody or antibody fragment that includes a humanized anti-CD19 binding domain, a humanized anti-CD22 binding domain, a humanized anti-CD20 binding domain or a humanized anti-BCMA binding domain, a transmembrane domain, and one or more cytoplasmic co-stimulatory signalling domains. The population of cells may comprise a population of cells expressing a CAR, wherein the CAR comprises an extracellular antibody or antibody fragment that includes two or more binding domains, such as a humanized anti-CD19 binding domain, a humanized anti-CD22 binding domain, a humanized anti-CD20 binding domain, and/or a humanized anti-BCMA binding domain, and a transmembrane domain and one or more cytoplasmic co-stimulatory signalling domains.

In certain embodiments of the invention, the population of cells administered as the adoptive cell transfer immunotherapy express T-cell receptors (TCRs). TCRs are antigen-specific molecules that are responsible for recognizing antigenic peptides presented in the context of a product of the major histocompatibility complex (MHC) on the surface of antigen presenting cells or any nucleated cell (e.g., all human cells in the body, except red blood cells). In contrast, antibodies typically recognize soluble or cell-surface antigens, and do not require presentation of the antigen by an MHC. This system endows T-cells, via their TCRs, with the potential ability to recognize the entire array of intracellular antigens expressed by a cell (including virus proteins) that are processed intracellularly into short peptides, bound to an intracellular MHC molecule, and delivered to the surface as a peptide-MHC complex. This system allows virtually any foreign protein (e.g., mutated cancer antigen or virus protein) or aberrantly expressed protein to serve as a target for T-cells.

In certain embodiments, in particular for treating autoimmune conditions, the adoptive cell transfer immunotherapy comprises administration of regulatory T-cells. In certain such embodiments, the cells express a construct targeting an antigen in the tissue that is inflamed. For example, in methods of treating multiple sclerosis, the cell targets an antigen present in the brain such as myelin oligodendrocyte glycoprotein (MOG), or for methods of treating Crohn's disease, the targeted antigen is generally expressed in the gut. The regulatory T-cells therefore target the inflamed tissue and reduce inflammation.

In further embodiments, the method uses a T-cell that targets a B-cell population that produces deleterious antibodies. In certain embodiments, the cell used is a chimeric autoantibody receptor T (CAAR-T) cell. In such embodiments, the cell expresses a construct presenting an antigen that is recognised by the problematic B-cells, such as a drug antigen, or an antigen from Table A, and the B-cell is eliminated upon binding the therapeutic cell. Such cells are also useful for treating autoimmune conditions and other conditions caused by B-cells producing deleterious antibodies.

According to certain aspects of the present invention, the engineered CAR cell may be allogeneic from a healthy donor and be further engineered to ablate or replace the endogenous TCR by gene editing technology such as CRISPR/cas9, ZFN, or TALEN, wherein the deletion of the endogenous TCR serves to eliminate CAR driven graft-versus-host disease.

According to certain aspects of the present invention, autologous cells (e.g., T-cell or NK-cells or dendritic cells) may be collected from the subject. These cells may be obtained from a number of sources, including peripheral blood mononuclear cells, bone marrow, lymph node tissue, cord blood, thymus tissue, tissue from a site of infection, ascites, pleural effusion, spleen tissue, and tumors. According to certain aspects of the present invention, allogeneic or xenogeneic cells may be used, typically isolated from healthy donors. When the T-cells, NK cells, dendritic cells, or pluripotent stem cells are allogeneic or xenogeneic cells, any number of cell lines available in the art may be used.

The cells can be obtained from a unit of blood collected from a subject using any number of techniques known to the skilled artisan, such as Ficoll^{™} separation. According to certain aspects of the present invention, cells from the circulating blood of an individual may be obtained by apheresis. The apheresis product typically contains lymphocytes, including T-cells, B-cells, monocytes, granulocytes, other nucleated white blood cells, red blood cells, and platelets.

Enrichment of a cell population by negative selection can be accomplished with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail typically includes antibodies to CD14, CD20, CD1 1b, CD16, HLA-DR, and CD8. According to certain aspects of the present invention, it may be desirable to enrich for or positively select for a cell population. For example, positive enrichment for a regulatory T-cell may use positive selection for CD4+, CD25+, CD62Lhi, GITR+, and FoxP3+.

The collected cells may be engineered to express the CAR or TCR by any of a number of methods known in the art. Moreover, the engineered cells may be expanded by any of a number of methods known in the art. As detailed above, the CAR or TCR may be bispecific, trispecific, or quadraspecific; the CAR or TCR may include a switch such as a goCAR or goTCR, or a safety switch CAR or TCR; the CAR or TCR may express immune-modulatory proteins such as an armored CAR or TCR.

According to certain aspects of the present invention, the collection of blood samples or apheresis product from a subject may be at any time period prior to when the expanded cells as described herein might be needed. As such, the source of the cells to be engineered and expanded (or simply expanded in the case of TILs) can be collected at any time point necessary, and desired cells, such as T-cells, NK-cells, dendritic cells, or TILs, can be isolated and frozen for later use in ACT, such as those ACT described herein.

According to certain aspects of the present invention, the population of cells expressing the CAR/TCR may be administered to the subject by dose fractionation, wherein a first percentage of a total dose is administered on a first day of treatment, a second percentage of the total dose is administered on a subsequent day of treatment, and optionally, a third percentage of the total dose is administered on a yet subsequent day of treatment.

An exemplary total dose comprises 10³ to 10¹¹ cells/kg body weight of the subject, such as 10³ to 10¹⁰ cells/kg body weight, or 10³ to 10⁹ cells/kg body weight of the subject, or 10³ to 10⁸ cells/kg body weight of the subject, or 10³ to 10⁷ cells/kg body weight of the subject, or 10³ to 10⁶ cells/kg body weight of the subject, or 10³ to 10⁵ cells/kg body weight of the subject. Moreover, an exemplary total dose comprises 10⁴ to 10¹¹ cells/kg body weight of the subject, such as 10⁵ to 10¹¹ cells/kg body weight, or 10⁶ to 10¹¹ cells/kg body weight of the subject, or 10⁷ to 10¹¹ cells/kg body weight of the subject.

An exemplary total dose may be administered based on a patient body surface area rather than the body weight. As such, the total dose may include 10³ to 10¹³ cells per m².

An exemplary dose may be based on a flat or fixed dosing schedule rather than on body weight or body surface area. Flat-fixed dosing may avoid potential dose calculation mistakes. Additionally, genotyping and phenotyping strategies, and therapeutic drug monitoring, may be used to calculate the proper dose. That is, dosing may be based on a patient's immune repertoire of immunosuppressive cells (e.g., regulatory T cells, myeloid-derived suppressor cells), and/or disease burden. As such, the total dose may include 10³ to 10¹³ total cells.

According to certain aspects of the present invention, cells may be obtained from a subject directly following a treatment. In this regard, it has been observed that following certain cancer treatments, in particular treatments with drugs that damage the immune system, shortly after treatment during the period when subjects would normally be recovering from the treatment, the quality of certain cells (e.g., T-cells) obtained may be optimal or improved for their ability to expand *ex vivo.* Likewise, following *ex vivo* manipulation using the methods described herein, these cells may be in a preferred state for enhanced engraftment and in vivo expansion. Thus, it is contemplated within the context of the present invention to collect blood cells, including T-cells, NK-cells, dendritic cells, or other cells of the hematopoietic lineage, during this recovery phase.

According to certain aspects of the present invention, the second dose may be the same or a different effective amount of a different population of cells expressing the same or a different CAR/TCR. Differences in the CAR/TCR may be in any aspect of the CAR/TCR such as, for example, different binding or antigen recognition domains or co-stimulatory domains. The second dose may additionally or alternatively include secreting cells with IL-12 or may even include adjuvant immunotherapies with small molecule inhibitors such as BTK, P13K, IDO inhibitors either concurrent or sequential to the cell therapy infusion.

According to certain aspects of the present invention, the methods may also comprise administration of one or more additional therapeutic agents, in addition to the adoptive cell transfer immunotherapy and the IgG cysteine protease or endoglycosidase. Exemplary therapeutic agents include a chemotherapeutic agent, an anti-inflammatory agent, an immunosuppressive, an immunomodulatory agent, or a combination thereof.

Therapeutic agents may be administered according to any standard dose regime known in the field. Exemplary chemotherapeutic agents include anti-mitotic agent, such as taxanes, for instance docetaxel, and paclitaxel, and vinca alkaloids, for instance vindesine, vincristine, vinblastine, and vinorelbine. Exemplary chemotherapeutic agents include a topoisomerase inhibitor, such as topotecan.

Exemplary chemotherapeutic agents include a growth factor inhibitor, a tyrosine kinase inhibitor, a histone deacetylase inhibitor, a P38a MAP kinase inhibitor, inhibitors of angiogenesis, neovascularization, and/or other vascularization, a colony stimulating factor, an erythropoietic agent, an anti-anergic agents, an immunosuppressive and/or immunomodulatory agent, a virus, viral proteins, immune checkpoint inhibitors, BCR inhibitors (e.g., BTK, P13K, etc.), immune-metabolic agents (e.g., IDO, arginase, glutaminase inhibitors, etc.), and the like. According to certain aspects of the present invention, the one or more therapeutic agents may comprise an antimyeloma agent. Exemplary antimyeloma agents include dexamethasone, melphalan, doxorubicin, bortezomib, lenalidomide, prednisone, carmustine, etoposide, cisplatin, vincristine, cyclophosphamide, and thalidomide, several of which are indicated above as chemotherapeutic agents, anti-inflammatory agents, or immunosuppressive agents.

### Cancers to be treated

The methods of the invention may improve the treatment of any cancer that may be treated using an adoptive cell transfer immunotherapy. In certain embodiments, the cancer is a blood cancer, which is a cancer that originates in blood-forming tissue, such as the bone marrow or other cells of the immune system. Treatment of blood cancers with adoptive cell transfer immunotherapies is well established and will benefit from the methods of the invention. Hematologic malignancies include, without limitation, leukemias (such as acute myeloid leukemia (AML), acute promyelocytic leukemia, acute lymphoblastic leukemia (ALL), acute mixed lineage leukemia, chronic myeloid leukemia (CML), chronic lymphocytic leukemia (CLL), hairy cell leukemia and large granular lymphocytic leukemia), myelodysplastic syndrome (MDS), myeloproliferative disorders (polycythemia vera, essential thrombocytosis, primary myelofibrosis and CML), lymphomas, multiple myeloma, monoclonal gammopathy of undetermined significance (MGUS) and similar disorders, Hodgkin's lymphoma, non-Hodgkin lymphoma (NHL), primary mediastinal large B-cell lymphoma, diffuse large B-cell lymphoma, follicular lymphoma, transformed follicular lymphoma, splenic marginal zone lymphoma, lymphocytic lymphoma, T-cell lymphoma, and other B-cell malignancies.

In certain embodiments, the cancer to be treated is a B-cell lymphoma. In certain embodiments, the method of the invention comprises multiple administrations of the IgG cysteine protease or IgG endoglycosidase and the cancer to be treated is a B-cell lymphoma. In certain such embodiments, the method of treating cancer comprises administering a protein that has IgG cysteine protease or IgG endoglycosidase activity after administering two or more doses of an adoptive cell transfer immunotherapy, which preferably comprises administration of CAR-T cells expressing CD19. In certain such embodiments, the method also includes immunosuppression. The invention may be particularly effective for treating tumours that require immunosuppression.

In further embodiments, the invention is for use in treating a solid cancer. Treatments with adoptive cell immunotherapies involving administration of fewer cells may be at increased risk of damaging effects of ADA. Also, treatment of solid cancers with adoptive cell immunotherapies is less efficacious, so may benefit greatly from the methods of the invention. "Solid cancers" include, without limitation, bone cancer, breast cancer, pancreatic cancer, skin cancer, cancer of the head or neck, cutaneous or intraocular malignant melanoma, uterine cancer, ovarian cancer, prostate cancer, rectal cancer, cancer of the anal region, colon cancer, stomach cancer, testicular cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, pediatric tumors, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, glioblastoma, pituitary adenoma, Kaposi's sarcoma, epidermoid cancer, squamous cell cancer, environmentally-induced cancers including those induced by asbestos. Solid cancers that are currently the subject of clinical trials using adoptive cell immunotherapies include neuroblastoma, glioblastoma, CNS tumor (in particular recurrent or refractory HER2 positive), sarcoma (in particular HER2-positive), osteosarcoma (in particular metastatic HER2-positive), liver tumors (in particular GPC3-positive Pediatric tumors), and the methods of the invention may be useful for treating these cancers.

In preferred embodiments, the uses of the invention are for treating cancer in patients that are not B-cell compromised. In preferred embodiments, the uses of the invention are for treating cancer in patients that are not immunosuppressed. Such patients may have more severe ADA.

Preferably, the uses of the invention are used for treating human patients. Where the subject is human, the subject can be of any age. For example, the subject can be 60 years or older, 65 or older, 70 or older, 75 or older, 80 or older, 85 or older, or 90 or older. Alternatively, the subject can be 60 years or younger, 55 or younger, 50 or younger, 45 or younger, 40 or younger, 35 or younger, 30 or younger, 25 or younger, or 20 or younger. For a human subject afflicted with cancer, the subject can be newly diagnosed, or relapsed and/or refractory, or in remission.

### Autoimmune conditions to be treated

The uses of the invention may improve the treatment of any autoimmune condition that may be treated using an adoptive cell transfer immunotherapy. In preferred embodiments, the autoimmune condition is selected from the list consisting of rheumatoid arthritis, systemic lupus erythematosus (lupus) inflammatory bowel disease (IBD), such as Crohn's disease or ulcerative colitis, multiple sclerosis (MS), Type 1 diabetes mellitus, Guillain-Barre syndrome, chronic inflammatory demyelinating polyneuropathy, psoriasis, Graves' disease, Hashimoto's thyroiditis, myasthenia gravis and vasculitis.

Table A below sets out further disease that may be treated according to the invention and the associated antigens. Methods of the invention may be used to treat any of these diseases or conditions.

**Table A**

| **DISEASE** | **AUTOANTIGENS** |
|---|---|
| ABO incompatible transplantation (Treatment method for End Stage Renal Disease (ESRD; stage 5 CKD)). | ABO erythrocyte antigens |
| Addison's disease | Steroid 21-hydroxylase, 17 alpha-Hydroxylase (17OH) and side-chain-cleavage enzyme (P450scc), Thyroperoxidase, thyroglobulin and H+/K(+)- |
| Anti-GBM glomerulonephritis (related to Goodpasture) | Anti-glomerular basement membrane (anti-GBM): noncollagenous (NC1) domains of the alpha3alpha4alpha5(IV) collagen |
| Anti-neutrophil cytoplasmic antibody-associated vasculitis (ANCA associated vasculitis) (Wegener granulomatosis, Churg-Strauss syndrome, microscopic polyangiitis) | Myeloperoxidase, proteinase 3 |
| Anti-NMDAR Encephalitis | N-methyl-D-aspartate receptor (NMDAR) |
| Anti-phospholipid antibody syndrome (APS) and catastrophic APS | Negatively-charged phospholipids complexed with phospholipid binding plasma proteins (e.g. beta2GPI), cardiolipin, beta2-glycoprotein I, and (beta2GPI) |
| Autoimmune bullous skin diseases (Pemphigus). Pemphigus foliaceus (PF), fogo selvagem (FS) (endemic form), pemphigus vulgaris (PV) | IgG against keratinocytes. Specific target is desmoglein (Dsg) 1 (desmosomal Cadherins) |
| Autoimmune hemolytic anemia (AIHA) | Self-antigens on red-blood-cells |
| Autoimmune hepatitis (AIH) | Actin, antinuclear antibody (ANA), smooth muscle antibody (SMA), liver/kidney microsomal antibody (LKM-1), anti soluble liver antigen (SLA/LP) and anti-mitochondrial antibody (AMA), CYP2D6, CYP2C9-tienilic acid, UGT1A, CYP1A2, CYP2A6, CYP3A, CYP2E1, CYP11A1, CYP17 and CYP21 |
| Autoimmune neutropenia (AIN) | FcgRIIIb |
| Bullous pemphigoid (BP) | Hemidesmosomal proteins BP230 and BP180 (type XVII collagen), laminin 5, the alpha6 subunit of the integrin alpha6beta4 and p200 |
| Celiac disease | transglutaminase 2 (TG2), transglutaminase 3, actin, ganglioside, collagen, calreticulin and zonulin, thyroid, endocrine pancreas, anti-gastric and liver, anti-nuclear constituents, anti-reticulin, actin, smooth muscle, calreticulin, desmin, collagens, bone, anti-brain, ganglioside, neuronal, blood vessel |
| Chronic urticaria | Alpha-subunit of the high-affinity IgE receptor, IgE |
| Complete congenital heart block (CCHB) | Ro (Sjögren's syndrome antigen A (SSA)), La (Sjögens syndrome antigen B(SSB)) |
| Diabetes type 1A (T1DM) | Islet cell autoantibodies (ICA), antibodies to insulin (IAA), glutamic acid decarboxylase (GAA or GAD), protein tyrosine phosphatase (IA2 or ICA512), Insulinoma Associated Peptide-2. The number of antibodies, rather than the individual antibody, is thought to be most predictive of progression to overt diabetes. |
| Epidermolysis bullosa acquisita (EBA) | The 145-kDa noncollagenous aminoterminal (NC-1) domain of collagen VII |
| Essential mixed cryoglobulinemia | Essential mixed cryoglobulinemia antigens |
| Goodpasture's syndrome (also known as Goodpasture's disease and anti-glomerular basement membrane disease | alpha3(IV) collagen (=Goodpasture antigen) |
| Graves'disease (Basedow's disease), includes Goitre and hyperthyroidism, infiltrative exophthalmos and infiltrative dermopathy. | Thyrotropin receptor (TSHR) Thyroid peroxidase (TPO) |
| Guillain-Barré syndrome (GBS). Acute inflammatory demyelinating polyneuropathy (AIDP), acute motor axonal neuropathy (AMAN) | Gangliosides GM1, GM1b, GD1a, and GalNAc-GD1a, glycosphingolipid, myelin proteins PMP22 and P0 |
| Hemophilia - Acquired FVIII deficiency | Factor VIII |
| IgA nephritis | |
| Idiopathic thrombocytopenic purpura (ITP) | Platelet glycoprotein (GP) IIb-IIIa and/or GPIb-IX |
| Lambert-Eaton myasthenic syndrome (LEMS) | voltage gated calcium channels |
| Mixed Connective Tissue Disease (MCTD) | IgG directed against the spliceosome, U1-snRNP |
| Multiple Myeloma | Multiple Myeloma antigens |
| Myasthenia gravis Myasthenic crisis | Acetylcholine receptors (AchR), muscle-specific kinase (MuSK) |
| Myocarditis, dilated cardiomyopathy (DCM) (congestive cardiomyopathy) | heart-reactive autoantibodies against multiple antigens e.g. cardiac myosin |
| Neuromyelitis Optica (NMO) | Aquaporin 4 (AQP4) |
| Primary biliary cirrhosis (PBC) | pyruvate dehydrogenase complex (PDC)-E2 and other members of the oxaloacid dehydrogenase family, Glycoprotein-210, p62, sp100 |
| Primary Progressive Multiple Sclerosis (PPMS) | Myelin oligodendrocyte glycoprotein (MOG), Myelin proteolipid protein (PLP), |
| | transketolase (TK), cyclic nucleotide phosphodiesterase type I (CNPase I), |
| | collapsin response mediator protein 2, tubulin beta4, neurofascin |
| Rheumatic heart disease (RHD), (Rheumatic fever) | Cardiac myosin |
| Rheumatoid Arthritis (RA) | Type II collagen, citrullin (-ated proteins (e.g. (fibrinogen, vimentin, filaggrin, type II collagen, enolase)), G6PI, RFs (anti-Fc/IgG), Vimentin, and cytokeratin |
| Serum-sickness, immune complex hypersensitivity (type III) | Various antigens |
| Sjögren's Syndrome (SS) | Ro (Sjögens syndrome antigen A (SS-A)), La (Sjögens syndrome antigen B(SS-B)), p80 coilin, antinuclear antibodies, anti-thyroid, anti-centromere antibodies (Raynaud's phenomenon), anti-carbonic anhydrase II (distal renal tubular acidosis), anti-mitochondrial antibodies (liver pathology), cryoglobulins (evolution to non-Hodgkin's lymphoma). alpha- and beta-fodrin, islet cell autoantigen, poly(ADP)ribose polymerase (PARP), NuMA, Golgins, NOR-90, M3-muscarinic receptor |
| SLE including Lupus nephritis | Autoantibodies to nuclear constituents (e.g. dsDNA and nucleosomes), dsDNA, PARP, Sm, PCDA, rRNA Ribosome P proteins, C1q |
| Stiff-person syndrome (SPS) | glutamic acid decarboxylase (GAD), amphiphysin. |
| Systemic sclerosis (scleroderma) | DNA-topoisomerase I (Scl-70), U3 snRNP, U2 snRNP, 7-2 RNP, NOR-90, centromere-associated proteins, and nucleolar antigens, Anti-Th/To, Anti-RNA |
| | polymerase I/III, Anti-PDGF receptor, Anti-fibrillin-1, M3-muscarinic receptor, |
| Transplant rejection | Transplant rejection antigens |
| Thrombotic Thrombocytopenic Purpura (TTP) | ADAMTS13 |
| Wegener's granulomatosis (granulomatosis with polyangiitis) | |
| Anti-drug antibodies (ADA) | Any treatment which suffers from reduced efficacy due to the presence of antibodies specific for the therapeutic agent. Includes, for example, antibody-based therapeutics, gene therapy vectors, cell therapies including adoptive cell transfer (ACT) immunotherapies e.g. using CAR-T cells |

Preferably, the uses of the invention are used for treating human patients. Where the subject is human, the subject can be of any age. For example, the subject can be 60 years or older, 65 or older, 70 or older, 75 or older, 80 or older, 85 or older, or 90 or older. Alternatively, the subject can be 60 years or younger 50 years or younger, 45 or younger, 40 or younger, 35 or younger, 30 or younger, 25 or younger, or 20 or younger. For a human subject afflicted with an autoimmune condition, the subject can be newly diagnosed, or relapsed and/or refractory, or in remission.

### Infections to be treated

The uses of the invention may improve the treatment of any infection that may be treated using an adoptive cell transfer immunotherapy. In preferred embodiments, the infection is selected from the list consisting of human immunodeficiency virus (HIV), human T-lymphotropic virus (HTLV), hepatitis A (HAV), hepatitis B (HBV), hepatitis C (HCV), Epstein-Barr virus (EBV), human papillomavirus (HPV), Kaposi's sarcoma herpes virus (KSHV), Lassa virus, cytomegalovirus (CMV), coronavirus, such as COVID-19, *Aspergillus fumigatus* and tuberculosis. Preferably, the infection is a chronic infection. Preferably, the infection is HIV.

In such embodiments, the transferred cells will generally express a construct that recognises an antigen on the pathogen to be targeted. Exemplary antigens include: gag protein, p55, p24, p18, envelope glycoprotein (env), gp160, gp120, gp41, reverse transcriptase (pol), p66, and p31 of HIV; HAV core and surface antigens; HBV core and surface (S, M and L) antigens; HCV core and surface (E2, NS2, NS3, NS4 and NS5) antigens; EBVA, LMP2, EBNA1 and BZLF1 of EBV; E6, E7 of HPV; and pp65, immediate early (IE) antigen and IE1 of CMV.

### IgG cysteine proteases

The inventors have demonstrated that use of an IgG cysteine protease can protect cells and improve their survival, and so may be useful for treatment of cancer in combination with an adoptive cell transfer immunotherapy. The IgG cysteine protease for use with the invention is specific for IgG, which is the predominant class of antibodies in mammalian serum.

In preferred embodiments, the protease for use in the methods of the invention is imlifidase (IdeS) (Immunoglobulin G-degrading enzyme of *S. pyogenes).* IdeS is an extracellular cysteine protease produced by the human pathogen *S. pyogenes.* IdeS was originally isolated from a group A *Streptococcus* strain of serotype M1, but the *ides* gene has now been identified in all tested group A *Streptococcus* strains. IdeS has an extraordinarily high degree of substrate specificity, with its only identified substrate being IgG. IdeS catalyses a single proteolytic cleavage in the lower hinge region of the heavy chains of all subclasses of human IgG. IdeS also catalyses an equivalent cleavage of the heavy chains of some subclasses of IgG in various animals. IdeS efficiently cleaves IgG to Fc and F(ab')₂ fragments via a two-stage mechanism. In the first stage, one (first) heavy chain of IgG is cleaved to generate a single cleaved IgG (scIgG) molecule with a non-covalently bound Fc molecule. The scIgG molecule is effectively an intermediate product which retains the remaining (second) heavy chain of the original IgG molecule. In the second stage of the mechanism this second heavy chain is cleaved by IdeS to release a F(ab')₂ fragment and a homodimeric Fc fragment. These are the products generally observed under physiological conditions. Under reducing conditions the F(ab')₂ fragment may dissociate to two Fab fragments and the homodimeric Fc may dissociate into its component monomers. SEQ ID NO: 1 is the full sequence of IdeS including the N terminal methionine and signal sequence. It is also available as NCBI Reference sequence no. WP_010922160.1. SEQ ID NO: 2 is the mature sequence of IdeS, lacking the N terminal methionine and signal sequence. It is also available as Genbank accession no. ADF13949.1.

In alternative embodiments, the protease for use in the methods of the invention is IdeZ, which is an IgG cysteine protease produced by *Streptococcus equi ssp. Zooepidemicus,* a bacterium predominantly found in horses. SEQ ID NO: 3 is the full sequence of IdeZ including N terminal methionine and signal sequence. It is also available as NCBI Reference sequence no. WP_014622780.1. SEQ ID NO: 4 is the mature sequence of IdeZ, lacking the N terminal methionine and signal sequence.

In alternative embodiments, the protease for use in the methods of the invention is a hybrid IdeS/Z, such as that of SEQ ID NO: 5. The N terminus is based on IdeZ lacking the N terminal methionine and signal sequence.

In preferred embodiments, the protease for use in the invention may comprise or consist of SEQ ID NO: 2, 4 or 5. Proteases for use in the invention may comprise an additional methionine (M) residue at the N terminus and/or a tag at the C terminus to assist with expression in and isolation from standard bacterial expression systems. Suitable tags include a histidine tag which may be joined directly to the C terminus of a polypeptide or joined indirectly by any suitable linker sequence, such as 3, 4 or 5 glycine residues. The histidine tag typically consists of six histidine residues, although it can be longer than this, typically up to 7, 8, 9, 10 or 20 amino acids or shorter, for example 5, 4, 3, 2 or 1 amino acids.

In further preferred embodiments, the protease for use in the invention may comprise, consist essentially, or consist of the sequence of any one of SEQ ID NOs: 6 to 25. These sequences represent IdeS and IdeZ polypeptides with increased protease activity and/or reduced immunogenicity. Each of SEQ ID NOs: 6 to 25 may optionally include an additional methionine at the N terminus and/or a histidine tag at the C terminus. The histidine tag preferably consists of six histidine residues. The histidine tag is preferably linked to the C terminus by a linker of 3x glycine or 5x glycine residues.

In further preferred embodiments, the protease for use in the invention may comprise, consist essentially, or consist of the sequence of any one of SEQ ID NOs: 56 to 69. These sequences represent IdeS polypeptides with increased protease activity and/or reduced immunogenicity. Each of SEQ ID NOs: 56 to 69 may optionally include an additional methionine at the N terminus and/or a histidine tag at the C terminus. The histidine tag preferably consists of six histidine residues. The histidine tag is preferably linked to the C terminus by a linker of 3x glycine or 5x glycine residues.

In further preferred embodiments, the protease for use in the invention may comprise, consist essentially, or consist of the sequence of any one of SEQ ID NOs: 6 to 25, optionally with up to 3 (such as 1, 2 or 3) amino acid substitutions. Each of SEQ ID NOs: 6 to 25 and variants thereof may optionally include an additional methionine at the N terminus and/or a histidine tag at the C terminus.

In further preferred embodiments, the protease for use in the invention may comprise, consist essentially, or consist of the sequence of any one of SEQ ID NOs: 56 to 69, optionally with up to 3 (such as 1, 2 or 3) amino acid substitutions. Each of SEQ ID NOs: 56 to 69 and variants thereof may optionally include an additional methionine at the N terminus and/or a histidine tag at the C terminus.

The polypeptide of the invention is typically at least 100, 150, 200, 250, 260, 270, 280, 290, 300 or 310 amino acids in length. The polypeptide of the invention is typically no larger than 400, 350, 340, 330, 320 or 315 amino acids in length. It will be appreciated that any of the above listed lower limits may be combined with any of the above listed upper limits to provide a range for the length the polypeptide of the invention. For example, the polypeptide may be 100 to 400 amino acids in length, or 250 to 350 amino acids in length. The polypeptide is preferably 290 to 320 amino acids in length, most preferably 300 to 315 amino acids in length.

The primary structure (amino acid sequence) of a protease of the invention is based on the primary structure of IdeS, IdeZ or IdeS/Z, specifically the amino acid sequence of SEQ ID NO: 2, 4 or 5, respectively. The sequence of a protease of the invention may comprise a variant of the amino acid sequence of SEQ ID NO: 2, 4 or 5, which is at least 80% identical to the amino acid sequence of SEQ ID NO: 2, 4 or 5. The variant sequence may be at least 80%, at least 85%, preferably at least 90%, at least 95%, at least 98% or at least 99% identical to the sequence of SEQ ID NO: 2, 4 or 5. The variant may be identical to the sequence of SEQ ID NO: 2, 4 or 5 apart from the inclusion of one or more of the specific modifications identified in WO2016/128558 or WO2016/128559. Identity relative to the sequence of SEQ ID NO: 2, 4 or 5 can be measured over a region of at least 50, at least 100, at least 200, at least 300 or more contiguous amino acids of the sequence shown in SEQ ID NO: 2, 4 or 5, or more preferably over the full length of SEQ ID NO: 4 or 5.

The protease for use in the invention may be an IdeS, IdeZ or IdeS/Z polypeptide that comprises a variant of the amino acid sequence of SEQ ID NO: 2, 4 or 5 in which modifications, such as amino acid additions, deletions or substitutions are made relative to the sequence of SEQ ID NO: 2, 4 or 5. Such modifications are preferably conservative amino acid substitutions. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art.

IgG cysteine protease activity may be assessed by any suitable method, for example by incubating a polypeptide with a sample containing IgG and determining the presence of IgG cleavage products. Suitable methods are described in the WO2016/128559. Suitable assays include an ELISA-based assay, such as that which is described in WO2016/128559. In such an assay, the wells of an assay plate will typically be coated with an antibody target, such as bovine serum albumin (BSA). Samples of the polypeptide to be tested are then added to the wells, followed by samples of target-specific antibody that is antibody specific for BSA in this example. The polypeptide and antibody are allowed to interact under conditions suitable for IgG cysteine protease activity. After a suitable interval, the assay plate will be washed and a detector antibody which specifically binds to the target-specific antibody will be added under conditions suitable for binding to the target-specific antibody. The detector antibody will bind to any intact target-specific antibody that has bound to the target in each well. After washing, the amount of detector antibody present in a well will be proportional to the amount of target-specific antibody bound to that well. The detector antibody may be conjugated directly or indirectly to a label or another reporter system (such as an enzyme), such that the amount of detector antibody remaining in each well can be determined. The higher the potency of the tested polypeptide that was in a well, the less intact target-specific antibody will remain and thus there will be less detector antibody. Typically, at least one well on a given assay plate will include IdeS instead of a polypeptide to be tested, so that the potency of the tested polypeptides may be directly compared to the potency of IdeS. IdeZ and IdeS/Z may also be included for comparison.

Other assays may determine the potency of a tested polypeptide by directly visualizing and/or quantifying the fragments of IgG which result from cleavage of IgG by a tested polypeptide. An assay of this type is also described in WO2016/128559. Such an assay will typically incubate a sample of IgG with a test polypeptide (or with one or more of IdeS, IdeZ and IdeS/Z as a control) at differing concentrations in a titration series. The products which result from incubation at each concentration are then separated using gel electrophoresis, for example by SDS-PAGE. Whole IgG and the fragments which result from cleavage of IgG can then be identified by size and quantified by the intensity of staining with a suitable dye. The greater the quantity of cleavage fragments, the greater the potency of a tested polypeptide at a given concentration. A polypeptide of the invention will typically produce detectable quantities of cleavage fragments at a lower concentration (a lower point in the titration series) than IdeZ and/or IdeS. This type of assay may also enable the identification of test polypeptides that are more effective at cleaving the first or the second heavy chain of an IgG molecule, as the quantities of the different fragments resulting from each cleavage event may also be determined. A polypeptide of the invention may be more effective at cleaving the first chain of an IgG molecule than the second, particularly when the IgG is an IgG2 isotype. A polypeptide of the invention may be more effective at cleaving IgG1 than IgG2.

### IgG endoglycosidases

The inventors have demonstrated that use of an IgG endoglycosidase can protect cells and improve their survival, and so may be useful for treatment of cancer in combination with an adoptive cell transfer immunotherapy. The IgG endoglycosidases for use with the invention are specific for IgG, which is the predominant class of antibodies in mammalian serum.

The agent may be a protein which has IgG endoglycosidase activity, preferably cleaving the glycan moiety at Asn-297 (Kabat numbering) in the Fc region of IgG. A preferred example of such a protein is EndoS (Endoglycosidase of *S. pyogenes*), which is shown to be effective in the examples. EndoS hydrolyzes the β-1,4-di-*N*-acetylchitobiose core of the asparagine-linked glycan of normally-glycosylated IgG (see Figure 18). The mature sequence of EndoS is provided as SEQ ID NO: 90. The protein may comprise or consist of the amino acid sequence of SEQ ID NO: 90, or may be a homologue thereof from an alternative bacterium, such as *Streptococcus equi* or *Streptococcus zooepidemicus,* or *Corynebacterium pseudotuberculosis, Enterococcus faecalis,* or *Elizabethkingia meningoseptica.* The agent may be CP40, EndoE, or EndoF₂.

Alternatively, the protein may be a variant of the EndoS protein which comprises or consists of any amino acid sequence which has at least 80%, 85%, 90% or 95% identity with SEQ ID NO: 90 and has IgG endoglycosidase activity. A variant of the EndoS protein may comprise or consist of an amino acid sequence in which up to 1, 2, 3, 4, 5, 10, 20, 30, 40, 50, 60, 70, 80, 90, or more, amino acid substitutions, insertions or deletions have been made relative to the amino acid sequence of SEQ ID NO: 90, provided the variant has IgG endoglycosidase activity. Said amino acid substitutions are preferably conservative.

Alternatively the agent may be a protein which comprises or consists of a fragment of SEQ ID NO: 90 and has IgG endoglycosidase activity, preferably wherein said fragment is 400 to 950, 500 to 950, 600 to 950, 700 to 950 or 800 to 950 amino acids in length. A preferred fragment consists of amino acids 1 to 409 of SEQ ID NO: 90, which corresponds to the enzymatically active α-domain of EndoS generated by cleavage by the streptococcal cysteine proteinase SpeB. The fragment may be created by the deletion of one or more amino acid residues of the amino acid sequence of SEQ ID NO: 90. Up to 1, 2, 3, 4, 5, 10, 20, 30, 40, 50, 100, 200, 300, 400, 500 or 550 residues may be deleted, or more. The deleted residues may be contiguous with other.

Any fragment or variant of SEQ ID NO: 2 preferably includes residues 191 to 199 of SEQ ID NO: 90, i.e. Leu-191, Asp-192, Gly-193, Leu-194, Asp-195, Val-196, Asp-197, Val-198 and Glu-199 of SEQ ID NO: 1. These amino acids constitute a perfect chitinase family 18 active site, ending with glutamic acid. The glutamic acid in the active site of chitinases is essential for enzymatic activity. Most preferably, therefore, a variant of SEQ ID NO: 90 contains Glu-199 of SEQ ID NO: 90. The variant of SEQ ID NO: 2 may contain residues 191 to 199 of SEQ ID NO: 90 having one or more conservative substitutions, provided that the variant contains Glu-199 of SEQ ID NO: 90.

### Assessment of IgG levels and timing of administrations

The appropriate timing of the administration of the protein that has IgG cysteine protease or IgG endoglycosidase activity and the adoptive cell transfer immunotherapy in the methods of the invention can be determined using, for example, assays for assessing serum IgG levels. For example, the amount of time it takes the protein to inactivate or eliminate Fc receptor and/or complement binding by substantially all IgG molecules present in the serum of the subject can be measured. This may optionally be determined by testing a serum sample taken from the individual and applying any suitable assay. Some exemplary suitable assays are described in the Examples.

Such an assay may directly test for the presence of IgG molecules in a serum sample that are able to bind to one or more Fc receptors, for example in an ELISA. Alternatively, such an assay may be indirect, in that it may test for the presence of one or more reaction products that are expected to result from the treatment of IgG with the protein that has IgG cysteine protease or IgG endoglycosidase activity. For example, where the agent is an enzyme which cleaves the IgG protein, a serum sample may be assayed for the presence of intact IgG molecules or the fragments which result from cleavage. This may be achieved by any suitable method, such as by separating the molecules and fragments based on molecular weight, e.g. by mass spectrometry or SDS-PAGE, or by specific detection of the molecules or fragments, e.g. by ELISA. Alternatively IgG may be detected by mixing serum from a subject with cells expressing FcgR's and monitoring IgG binding by flow cytometry using fluorochrome conjugated anti-human IgG.

Conventional methods for assessing the quantity of IgG in a sample, such as a serum sample, in a clinical setting rely on nephelometry and turbidimetry because of their speed, ease of use and precision. In both nephelometry and turbidimetry, a light source is projected through a liquid sample within a transparent container. Turbidimetry measures the decrease in the intensity of light and nephelometry measures scatter of light as it passes through the sample, which is proportional to the concentration of the immunoglobulin in the solution. Both principles are based on added anti IgG antibodies that react with antigen in the sample to form an antigen/antibody complex (agglutination). Addition of PEG allows the reaction to progress rapidly to the end point, increases sensitivity, and reduces the risk of samples containing excess antigen producing false negative results. In the case of IgG analysis, the F(ab')₂-part of IgG is cross-linked by the anti-IgG antibody and cause the agglutination reaction. However, such methods may not be appropriate when some or all of the IgG present may not be intact. For example, if an IgG cysteine protease (such as IdeS) has been administered to the subject from whom the sample is taken, e.g. in a method of the invention, or if such a protease has been administered to the sample, cleavage fragments such as F(ab')₂- and Fc-fragments will be present. This does not affect the agglutination reaction of conventional nephelometry and turbidimetry methods as long as the F(ab')₂ fragments are still present in the sample. Due to the shorter half-life of F(ab')₂ fragments compared to intact IgG, the agglutination will decrease over time though it is not proportional to the amount of intact IgG present in the sample. Thus, samples affected by the presence of an IgG cysteine protease (such as IdeS) cannot be assessed by conventional methods. The inventors developed a new assay for IgG concentration which is compatible with samples affected by the presence of an IgG cysteine protease (such as IdeS) and may be used in any clinical setting, including (but not limited to) uses in combination with other methods of the invention.

Said method is able to discriminate between intact IgG and IdeS-generated F(ab')₂-fragments. This was accomplished by making use of antibodies that detect the different fragments i.e. an anti-Fab antibody and an anti-Fc antibody. The antibodies used in the assay must not be a substrate for the IgG cysteine protease affecting the sample (typically IdeS). This avoids the assay reagents being affected by any active protease which may be present in a sample. This can be accomplished by testing IgG from different species or by using antibody fragments (i.e. Fab fragments or F(ab')₂ fragments) in place of whole antibodies. Typically, an anti-F(ab')₂ agent is incubated with the sample as a capture reagent. The capture reagent is typically immobilized, for example in the wells of an assay plate. Bound IgG is then detected by incubation with an anti-Fc agent as the detector reagent. Thus, only IgG which possess both Fab and Fc parts will be detected, contrary to the nephelometry and turbidimetry methods. The detector reagent may typically be conjugated directly or indirectly to a moiety to facilitate detection, such as a fluorescent dye or an enzyme which reacts with a chromogenic substrate. The capture and detector reagents can be any other molecule that specifically recognizes the Fab- or Fc-part of IgG and can be used in the reverse order i.e. capture using anti-Fc and detect using anti-Fab. The assay may be conducted in any suitable format, such as a conventional ELISA or Meso Scale Discovery format.

In some cases, such as when the IgG cysteine protease is IdeS, the sample may include intermediate fragments such as scIgG in which only one heavy chain is cleaved, and the F(ab')₂ remains attached to the other, intact heavy chain. In such cases, the scIgG fragment may be incorrectly identified by the assay as an intact IgG. Thus, the method may include a complimentary step of assessing the sizes of the fragments present in the sample. Since there are no disulphide bridges between the heavy chains below the hinge region, the Fc-part of the heavy chain in a scIgG fragment will separate from the intact heavy-chain under denaturing conditions as an approximately 20-25 kDa protein. The different fragment sizes can be detected and quantified using any suitable method, such as SDS-PAGE. A specific embodiment of the method, including the optional complimentary step is described in Example 1 (see *Efficacy assessment*). The method is particularly useful for assessing the efficacy of IdeS in a clinical setting.

Where the protein that has IgG cysteine protease or IgG endoglycosidase activity is an enzyme which cleaves a glycan moiety on IgG, a serum sample may be assayed for the presence of IgG molecules which possess either normal or truncated glycans, or for the glycan fragments that result from cleavage. This may be achieved by any suitable method, such as by separating the molecules and/or fragments based on molecular weight, e.g. by mass spectrometry or SDS-PAGE, or by specific detection of the molecules or fragments, e.g. by ELISA.

In methods wherein the protein that has IgG cysteine protease or IgG endoglycosidase activity is administered before the adoptive cell transfer immunotherapy, the lower limit of the time interval between administration of the protein that has IgG cysteine protease or IgG endoglycosidase activity and the adoptive cell transfer immunotherapy may be selected from: at least 30 minutes, at least 1 hour, at least 2 hours, at least 3 hours, at least 4 hours, at least 5 hours, or at least 6 hours. The lower limit may be shorter than any of the above should it be determined that Fc receptor binding by substantially all IgG molecules present in the serum of the subject has been sufficiently reduced or eliminated at an earlier time point. Preferably, the lower limit is 2 hours.

In methods wherein the protein that has IgG cysteine protease or IgG endoglycosidase activity is administered before the adoptive cell transfer immunotherapy, the upper limit of the time interval between administration of the protein that has IgG cysteine protease or IgG endoglycosidase activity and the adoptive cell transfer immunotherapy may be selected independently of the lower limit, and may be determined by the time that it takes for endogenous production of IgG to begin to replace or to completely replace the IgG molecules that were present in the serum of the subject prior to carrying out the method. This may be determined by testing a serum sample taken from the individual and applying any suitable assay, such as those described above with respect to the lower limit. Newly-synthesised IgG typically starts to reappear in serum within 3-4 days, with total replacement complete by around 3 weeks (21 days). Therefore, the upper limit may be selected from: at most 21 days, at most 18 days, at most 14 days, at most 13 days, at most 12 days, at most 11 days, at most 10 days, at most 9 days, at most 8 days, at most 7 days, at most 6 days, at most 5 days, at most 4 days, at most 3 days, at most 2 days, at most 24 hours, at most 18 hours, at most 12 hours, at most 10 hours, at most 8 hours, at most 7 hours, at most 6 hours, at most 5 hours, at most 4 hours, at most 3 hours, at most 2 hours, or at most 1 hour. Preferably, the upper limit is 48 hours.

The time interval between administration of the protein that has IgG cysteine protease or IgG endoglycosidase activity and the adoptive cell transfer immunotherapy may be at most 24 hours, at most 12 hours, or at most 6 hours, so that both administrations steps (a) and (b) may be carried out on the same day or during the same visit to a treatment centre. This is highly advantageous, particularly where access to treatments centres may be limited.

In methods wherein the protein that has IgG cysteine protease or IgG endoglycosidase activity is administered after the adoptive cell transfer immunotherapy in order to increase the efficacy of the immunotherapy, the timing of the administrations depends on the rise in IgG ADA. In certain embodiments, the protein is administered 4-8 days, such as 5-7 or 6 days, after the adoptive cell transfer immunotherapy. This timing may be appropriate if the antibody response is a recall response. If the antibody response is a primary response, the protein may be administered more than a week after the adoptive cell transfer immunotherapy, such as 10 days, 2 weeks, 3 weeks or 4 weeks, or between 10 days and 2 weeks, between 10 days and 3 weeks, between 2 and 4 weeks.

### Production of polypeptides

A polypeptide as disclosed herein may be produced by any suitable means. For example, the polypeptide may be synthesised directly using standard techniques known in the art, such as Fmoc solid phase chemistry, Boc solid phase chemistry or by solution phase peptide synthesis. Alternatively, a polypeptide may be produced by transforming a cell, typically a bacterial cell, with a nucleic acid molecule or vector which encodes said polypeptide. Production of polypeptides by expression in bacterial host cells is described and exemplified in WO2016/128559.

### Compositions and formulations comprising polypeptides

The present invention also provides compositions comprising an IgG cysteine protease or IgG endoglycosidase, for use in the therapeutic methods of the invention. For example, the invention provides a composition comprising one or more polypeptides of the invention, and at least one pharmaceutically acceptable carrier or diluent. The carrier (s) must be 'acceptable' in the sense of being compatible with the other ingredients of the composition and not deleterious to a subject to which the composition is administered. Typically, carriers and the final composition, are sterile and pyrogen free.

Formulation of a suitable composition can be carried out using standard pharmaceutical formulation chemistries and methodologies all of which are readily available to the reasonably skilled artisan. For example, the agent can be combined with one or more pharmaceutically acceptable excipients or vehicles. Auxiliary substances, such as wetting or emulsifying agents, pH buffering substances, reducing agents and the like, may be present in the excipient or vehicle. Suitable reducing agents include cysteine, thioglycerol, thioreducin, glutathione and the like. Excipients, vehicles and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethylene glycol, hyaluronic acid, glycerol, thioglycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients, vehicles and auxiliary substances is available in Remington's Pharmaceutical Sciences (Mack Pub. Co., N.J. 1991).

Such compositions may be prepared, packaged, or sold in a form suitable for bolus administration or for continuous administration. Injectable compositions may be prepared, packaged, or sold in unit dosage form, such as in ampoules or in multi-dose containers containing a preservative. Compositions include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of a composition for parenteral administration, the active ingredient is provided in dry (for e.g., a powder or granules) form for reconstitution with a suitable vehicle (e. g., sterile pyrogen-free water) prior to parenteral administration of the reconstituted composition. The compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono-or di-glycerides.

Other parentally-administrable compositions which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. The compositions may be suitable for administration by any suitable route including, for example, intradermal, subcutaneous, percutaneous, intramuscular, intra-arterial, intraperitoneal, intraarticular, intraosseous or other appropriate administration routes. Preferred compositions are suitable for administration by intravenous infusion.

### General

In addition as used in this specification and the appended claims, the singular forms "a", "an", and "the" include plural referents unless the content clearly dictates otherwise. Thus, for example, reference to "a polypeptide" includes "polypeptides", and the like.

Unless specifically prohibited, the steps of a method disclosed herein may be performed in any appropriate order and the order in which the steps are listed should not be considered limiting.

A "polypeptide" is used herein in its broadest sense to refer to a compound of two or more subunit amino acids, amino acid analogs, or other peptidomimetics. The term "polypeptide" thus includes short peptide sequences and also longer polypeptides and proteins. As used herein, the term "amino acid" refers to either natural and/or unnatural or synthetic amino acids, including both D or L optical isomers, and amino acid analogs and peptidomimetics.

The terms "patient" and "subject" are used interchangeably and typically refer to a human. References to IgG typically refer to human IgG unless otherwise stated.

Amino acid identity as discussed above may be calculated using any suitable algorithm. For example the PILEUP and BLAST algorithms can be used to calculate identity or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10. Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001. Alternatively, the UWGCG Package provides the BESTFIT program which can be used to calculate identity (for example used on its default settings) (Devereux et al (1984) Nucleic Acids Research 12, 387-395).

### Examples

The following experiments were performed to investigate whether preexisting or treatment-induced antibodies have negative effects on CAR T-cells and if these effects can be mitigated through treatment with imlifidase or EndoS. Systems were developed to mimic anti-drug antibodies binding to CAR T-cells by employing *in vitro* and *in vivo* models where IgG bound to cell-surface receptors. These models mimic antibody-mediated effector mechanisms of surface-bound antibodies on chimeric antigen receptors. The effect of treatment with imlifidase or EndoS in the model systems was investigated.

Unless indicated otherwise, the methods used are standard biochemistry and molecular biology techniques. Examples of suitable methodology textbooks include Sambrook et al., Molecular Cloning, A Laboratory Manual (1989) and Ausubel et al., Current Protocols in Molecular Biology (1995), John Wiley and Sons, Inc.

### Material and Methods - Examples 1-4

### Animals and cell lines

Balb/c JBomTac, 6-week-old females were purchased from Taconic and acclimatized before use at 9 weeks of age. The mice were housed and treated in accordance with ethical approval M72-13 (Lund).

Daudi cells (ACC78) and THP1 monocytic cell line (AML; TIB-202) were cultured in complete D-MEM 10 medium (Glutamax D-MEM, 5% FCS and PEST).

### Antibodies and complement

Rabbit anti-mouse thrombocyte IgG (serum) (Code #CLA31440, lot 6327, Cederland) was purified with protein G. The purified IgG was treated with IdeS to generate scIgG and fully-cleaved Fc/F(ab')2 fractions for the immune thrombocytopenia (ITP) experiments. The purity of the cleavage products was confirmed by SDS-page gel analysis.

Anti-CD20 IgG (Rituximab, N7022, 10 mg/mL) was purchased from Mabthera and human IgG1 isotype negative control (# I5154) was purchased from Sigma.

CCK-8, a cell counting kit from Dojindo Laboratories, Japan, was used according to instructions.

Baby rabbit serum complement (CL3441; lot 6374; Cedarlane) was reconstituted with sterile water before use and diluted in medium at a final dilution of 1:10.

Human blood was collected in BD CAT tubes (#368815; Spray dried clot activator (silica, PVP, L-720)) for serum and frozen at -20°C. Serum was tested for low toxicity on Daudi cells before use as complement source.

Mouse anti-human C4d (A213, Quidel) was biotinylated in-house and used at 1µg/ml for cell staining.

Polyclonal sheep Anti-human C1q (MD-14-0162, Raybiotech) was fractionated into F(ab')2s and purified using the FragIT kit (A2-FR2-025, Genovis) (final staining conc. 75 µg/ml). Donkey anti-sheep IgG(H+L)-bio (Jackson, 713-065-003) was used as detection antibody (2,5 µg/ml) together with fluorochrome SA-PE (BD-Pharmingen, #554061).

7-AAD (Sigma, A9400) was used as dead cell marker in CDC assays.

For the ADCP assay, THP1 cells were stained with FarRed DDAO-SE (Molecular Probes, C34553, Lot: 33C1-1) 1mg/ml in DMSO (approx. 2mM) and Daudi cells with calcein, AM (Invitrogen, C3099, Lot 25257W); (1mg/ml stock solution in DMSO).

### Enzymes

His-tagged-EndoS from *Streptococcus pyogenes* was expressed in *E. coli* and purified. Lipopolysaccharide (LPS) was removed using the EndoTrap blue matrix. Purity was controlled on SDS-page gels. Additional protein bands after His-tag purification beyond the expected 100kD band have been shown not to be host cell proteins but of EndoS origin by mass spectrometry.

IdeS (IgG-degrading enzyme of *Streptococcus pyogenes*) is a secreted cysteine endopeptidase from the human pathogen *S. pyogenes* that catalyzes with a high degree of specificity a single proteolytic cleavage at the lower hinge of human immunoglobulin G (IgG) antibodies.

### Example 1 - Antibody-mediated complement binding on target cells

The effect of preexisting or treatment-induced antibodies on cell therapies and the impact of IdeS and EndoS on such antibodies was investigated using CD20-positive Daudi cells opsonized with rituximab (RTX), which is a model system for ADA interactions in CAR-T therapies in the sense that a receptor-specific antibody (RTX) binds to a cell surface receptor (CD20) in analogy to an ADA binding to a CAR.

### Complement deposition on Daudi cells

Daudi cells (50 µl at 3 x 10E7/ml) were treated with anti-CD20 antibody RTX or human IgG1 negative control antibodies at a final concentration of 2 µg/ml together with a 1:10 step titration of either IdeS or EndoS, starting at 100 µg/ml down to a final conc. of 0.01 µg/ml enzymes. The 96 V-well master-plate containing a total volume of 150 µl/well was incubated at 37°C for 110 min. The plate was centrifuged and a volume of 50 ul supernatant was removed. 100 µl complement was added in form of human serum at a dilution of 1:5 resulting in a final serum dilution of 1:10. The plate was incubated for 120 min at 37°C. All dilutions and washing steps were made in D-MEM complemented with 0.5% BSA.

### Flowcytometry staining for complement deposition on cells

C1q complement-binding was assessed through incubation with sheep-anti-C1q F(ab')2 generated form polyclonal IgG (# MD-14-0162; RayBiotech) which was previously cleaved into Fc and F(ab')2 fragments using resin-bound IdeS ( FragIT kit, # A0-FR6; Genovis). Fc fragments were removed with protein A column. The polyclonal F(ab')2 was used at a final concentration of 75 µg/ml). A biotinylated Donkey-anti-Sheep IgG (H+L) (#713-065-003, Jackson ImmunoResearch) was used to detect the sheep-anti human C1q F(ab')2. SA-PE was diluted in D-MEM (+0,5% BSA) and used as detection fluorochrome.

C4d complement binding was assessed through incubation with a biotinylated mouse -anti-human C4d antibody (# A213; Quidel). SA-PE was diluted in D-MEM (0,5% BSA) and used as detection fluorochrome. Finally, cells were transferred to FACS tubes and analysed for mean fluorescent intensity (MFI) in FL2 using an Accuri C6 cytofluorometer.

### Results

Complement deposition on IgG binding to target cells can initiate the classical complement pathway, which can lead to the formation of a membrane attack complex (MAC) resulting in cell death. Earlier stages in the complement cascade, facilitated through antibody binding, can also flag cells for complement-mediated phagocytosis via complement receptors. Pre-existing and induced antibodies against CAR T-cells can therefore limit CAR-T persistence in patients.

Complement deposition of IgG-coated cells was investigated using CD20-positive Daudi cells opsonized with RTX and thereafter incubated with indicated concentrations of either IdeS or EndoS. Two hours later human serum was added as complement source for an additional two hours. Cells were aliquoted and stained using anti-human C1q- or C4d-specific detection antibodies and SA-PE as fluorochrome. Cells were analysed for MFI by single cell flowcytometry.

Both C1q and C4d deposition by IgG can be prevented by IdeS and EndoS (see Figure 1). This suggests that IgG sensitized CAR T-cells will also be protected from complement deposition by IdeS or EndoS treatment.

### Example 2 - Complement-dependent cytotoxicity (CDC)

The binding of antibodies to receptor molecules, including preexisting or induced CAR-specific antibodies, can result in full complement activation leading to the formation of the cytotoxic MAC. To mimic this, Daudi cells were incubated with the CD20-receptor specific antibody RTX and it was investigated if CDC could be prevented through the treatment with IdeS or EndoS.

Daudi cells (3x10E6) were incubated for 60 min with IdeS or EndoS (50 µg/ml) together with titrated concentrations of rituximab (RTX) (in well conc. 50 µg/ml, 1:2 step dilutions down to 0,2 µg/ml) in a master plate for 60 min at 37°C.

50 µl of the cell mix was transferred to ELISA plates together with 50 ul baby rabbit serum (1:5 dilution) as a complement source and incubated for 45 min at 37°C for CDC lysis. 10 ul CCK8 was added to each 100 ul mix and incubated at 37°C in the CO2-incubator for an additional 60 min. HCl (50 ul, 0,1M) was used as stop-solution. OD-values were acquired at 450 nm. OD values from wells without RTX were set as 100% survival.

### Results

At an RTX concentration of 0.78 µg/ml, 70 % of the cells in the medium group are dead while in the presence of IdeS or EndoS at this concentration all Daudi cells survive (see Figure 2). The protective effect of EndoS decreases with increasing RTX concentrations down to 30% survival at 50 µg/ml, nevertheless, this is a 60x higher RTX concentration than the same survival at 0,78 µg/ml without EndoS. Therefore, even at high antibody concentrations (50 µg/ml), CDC can be prevented with IdeS, whilst EndoS also exhibits potent protective activity.

### Example 3 - ADPC of RTX-opsonized Daudi cells by THP1 effector cells

These experiments addressed the question of whether IdeS and EndoS reduce FcyR-mediated phagocytosis (ADCP), which is one mechanism that could be limiting for CAR T-cell persistence in the presence of preexisting or induced CAR-specific antibodies.

### Cell culture

THP1 (monocytic effector cells) and Daudi target cells were expanded in complete Glutamax D-MEM (5% FCS and PEST), washed and diluted in D-MEM 10 medium before the experiments.

### Target Cell labelling

Daudi cells were washed twice in PBS to remove proteins before labelling. Cells were resuspended in PBS and stained using calcein (4 µL from 1 mg/mL to 6 mL cells) and incubated in the dark at RT. Cells were washed twice after 15 min in medium (0,5% BSA) and resuspended in 3 mL D-MEM 10 medium at 2 x 10⁶ cells/mL.

### Effector cell labelling

THP1 cells were washed twice in PBS to remove proteins and resuspended in 5 mL PBS. FarRed was added (5 µL to 5 mL cells) and incubated in the dark at RT. After 20 min the cells were washed twice in medium and resuspended in D-MEM 10 at 3,6 x 10⁶ cells/mL.

### Target-Effector cell incubation

Titrated RTX and enzymes (IdeS or EndoS) were incubated for 60 min at 37°C. Daudi target cells (50 µl at 2 x 10E6/ml) were incubated for 30 min to allow for opsonization before THP1 effector cells (50 µl at 3,6 x10E6/ml) were added and incubated for approximately 2h to allow for the ADCP of Daudi cells. Cells were fixed with 5% PFA for 3 min, washed and transferred for FACS analysis. Cells were analyzed using the Accuri C6 flow cytometer for MFI in FL2 (calcein) and FL4 (FarRed). Double positive cells were regarded as positive for ADCP.

### Results

The effectiveness of IdeS and EndoS to protect RTX-opsonized CD20-positive Daudi cells from FcyR-mediated phagocytosis by monocytic THP1 effector cells was analysed by flow cytometry. In short, calcein-labeled Daudi cells were opsonized with increasing concentrations of RTX before addition a fixed concentration of IdeS or EndoS. After an incubation at 37°C for 30 min FarRed-labeled THP1-effector cells were added for approximately two hours. Washed and fixed cells were then analysed by flow cytometry. All cells positive for calcein in FL2 were gated and defined as 100% Daudi cells. All FL2 positive cells were then gated in FL4 for RarRed positivity. These calcein/FarRed double positive cells are THP1-phagocytosed Daudi cells. The removal of the Fc part by IdeS fully abolished the ability of THP1 cells to phagocytize opsonized Daudi cells. The deglycosylation at position N297 of RTX by EndoS also resulted in a reduction of phagocytosis in this model (see Figure 3).

Therefore, in ADCP, the engulfment of antibody-opsonized cells by the monocytic cell line THP1 is fully prevented by IdeS treatment and is reduced by EndoS treatment.

### Example 4 - Protection of platelets in the immune thrombocytopenia (ITP) model

As demonstrated above using RTX and Daudi cells, IdeS and EndoS mitigate effector functions of cell-surface receptor-specific antibodies. The following experiment made use of a thrombocytopenia (ITP) model to investigate if these enzymes also protect antibody-sensitized endogenous cells from elimination *in vivo.* This demonstrates that deleterious polyclonal antibodies directed against cell surface receptors can be inactivated through the treatment with imlifidase or EndoS mimicking the effects of these enzymes in context of anti-CAR antibodies on the persistence of CAR T-cells.

### ITP in vivo EndoS treatment

Nine week old female Balb/c ^{JBomTac} mice were primed for immune thrombocytopenia (ITP) by a single 200 µl i.p. injection of anti-platelet specific antibodies (anti-PLT IgG) (50 µg IgG/mouse) purified from rabbit anti-mouse thrombocyte serum (Cederland #CLA31440) by Protein G. Indicated amounts of EndoS (10, 30, 90 µg /mouse) were injected i.p 30 min later, respectively PBS as negative control. Mice injected at both occasions with carrier solution (PBS) were used as normal controls. The mice were evaluated for hematoma or unusual behavior 4 hours after injection. Blood samples were taken from tail veins in Microvette CB300 (Sarstedt, Potassium-EDTA #16.444.100) after 24h. Platelets in blood samples were counted with the hematology analyzer VetScan HM5.

### ITP induction with in vitro IdeS cleaved anti-PLT antibodies

Anti-mouse PLT IgG was protein G-purified from serum (Code #CLA31440, Cederlane) of mouse thrombocyte immunized rabbits. IgGs were treated to produce the IdeS-cleavage products scIgG and fully cleaved Fc/F(ab')2 fragments for *in vivo* experiments.

Nine -week old female Balb/c ^{JBomTac} mice were primed for ITP by a single 200 µl i.p. injection of either 250µg/mouse anti-PLT IgG, scIgG or Fc/F(ab')2 fragments. Some mice were injected with PBS to establish normal control levels of thrombocytes. Blood samples were taken from tail veins in Microvette CB300 (Sarstedt, Potassium-EDTA #16.444.100) after 24h. Platelets in blood samples were counted with the hematology analyzer VetScan HM5.

### ITP in vivo IdeS treatment

Nine week old female Balb/c ^{JBomTac} mice were primed for ITP by a single 200 ul i.p. injection of anti-PLT IgG (250 µg IgG/mouse) purified from rabbit anti-mouse thrombocyte serum (Cederland #CLA31440) by Protein G. IdeS (0, 0.2, 2, 20 µg/mouse) was administered i.v. one hour post i.p. anti-PLT IgG injection. The control mice for normal thrombocyte levels were injected with PBS only. Blood samples were taken from the tail vein in Microvette CB300 (Sarstedt, Potassium-EDTA #16.444.100) after 24h. Platelets in blood samples were counted with the hematology analyzer VetScan HM5.

### Results

The aim with this study was to evaluate the effect in vivo of the IdeS-cleavage products scIgG or F(ab')2 IgG-fragments as mediators of effector function in comparison to intact anti-PLT IgG in vivo.

Immune thrombocytopenia (ITP) was induced in BALB/c mice by a single i.p. injection of either rabbit anti-mouse thrombocyte purified intact IgG, rabbit scIgG or F(ab')2 fragments at a dose of 0.25 mg/mouse. Mice were bled one day after the induction of ITP and blood was collected via the tail vein. Platelets were automatically counted in a VetScan HM5. Two naive mice received PBS only were used as control mice. They had a normal platelet level at 657 x 10⁹ platelets/L compared to mice injected with rabbit anti-mouse thrombocyte purified IgG that had a drop in platelet number down to 86 x 10⁹ platelets/L (see Figure 4). However, a small reduction of platelets could be seen in mice injected with anti-PLT scIgG. In mice treated with purified rabbit anti-mouse thrombocyte (F(ab')2 IgG-fragments, no induction of thrombocytopenia was observed.

From this experiment we can conclude that a protective effect from thrombocytopenia can be achieved through the *in vitro* generation of scIgG or (F(ab')2 by IdeS. An analogous protective effect can be expected from other preexisting or de novo antibodies targeting neoepitopes on chimeric antigen receptor T-cells.

The thrombocytopenia could be partly prevented when scIgG preparations were injected and fully abolished after the IgGs were cleaved by IdeS into Fc and F(ab')2 fragments (see Figure 4).

To investigate if IdeS also has a therapeutic effect *in vivo,* mice were first injected with a thrombocytopenic dose of anti-PLT rabbit IgG. One hour later different doses of IdeS were injected. 24 hours later blood was collected to establish platelets counts in the different groups. Two µg IdeS per mouse was fully sufficient to rescue normal levels of platelets in the mice (see Figure 5). The therapeutic effect of IdeS on deleterious anti-platelet antibodies (anti-PLT) has therefore been tested *in vivo* using an ITP mouse model. Injection of intact polyclonal anti-PLT rabbit antibodies (250 µg) into mice leads to a strong ITP phenotype with platelet depletion.

Similar results were seen when EndoS was injected into anti-PTL IgG sensitized mice. 50 µg anti-PLT antibodies were sufficient to reduce platelet counts from around 600x10E9/L to 200x10E9/L within 24h. Ten µg EndoS per mouse was sufficient to protect mice from thrombocytopenia (see Figure 6).

These experiments show that pathogenic antibody-mediated effector functions of opsonized cells can be prevented or ameliorated with the use of IdeS or EndoS. Based on these data, it is expected that these enzymes will also improve the survival and efficacy of CAR T/-NK cells through the inactivation of pre-existing and treatment-induced ADA.

In summary, it has been shown that the tested antibody-mediated effector functions (C1q- and C4d-deposition, CDC, ADCP), mimicking ADA responses against CAR-T cells, can be prevented through the use of IdeS and EndoS.

### Example 5

Experiments were performed to confirm if antibodies, directed towards the single chain variable fragment (scFv) chimeric antigen receptor or against allogeneic epitopes on CAR-T cells, have deleterious effects. CAR- and allo-specific antibodies from different sources were therefore tested for binding, and Fc-mediated antibody effector functions i.e., antibody-dependent cellular phagocytosis (ADCP) and antibody-dependent cell-mediated cytotoxicity (ADCC) of CAR-T cells. Furthermore, it was confirmed that imlifidase treatment can protect CAR-T cells from those deleterious IgG effector mechanisms.

### Material and Methods

### Cells

CAR-T cell line, anti-CD19scFv(FMC63)-h(28ζ)CAR-Jurkat T-cell (CARJ-ZP005, Creative biolabs, Shirley, NY, USA) based on clone E6-1; Jurkat wildtype (wt) (Clone E6-1) (#EP-CL-0129, ElabScience, Houston, TX, USA); Primary human CAR T cells, anti-BCMA4 CAR T-cell (BCMA-4-TM8-4-1BB-CD3zeta CAR-T Cells) (PM-CAR1037, ProMab Biotechnologies, Richmond, CA, USA); anti-CD19 CAR T-cell (CD19-scFv-Flag-TM--CD28-CD3zeta CAR-T Cells) (PM-CAR1007, ProMab Biotechnologies); Mock scFv control T cells (PM-CAR1000, ProMab Biotechnologies). The anti-CD19-scFv was derived from the murine CD19-specific monoclonal antibody FMC63.

THP-1 monocytic cell line (ACC16, DSMZ, Braunschweig, Germany). IgM BCR- and CD20-positive Daudi cells (ACC78, DSMZ). All cells were cultured in complete RPMI 10 medium (RPMI, 10% FCS and PEST). Anti-CD19 CAR-Jurkat T-cells were cultured under puromycin (1mg/ml) selection.

### Antibodies and human sera

CAR scFv cross-reactive anti-F(ab')₂-specific antibodies include affinity purified rabbit anti-human IgG, F(ab')₂ fragment specific (#309-005-006, JacksonImmunoResearch, West Grove, PA, USA), affinity purified rabbit anti-mouse IgG, F(ab')₂ fragment specific (#315-005-006, JacksonImmunoResearch), goat anti-human-IgG-Fc-PE (LS-AB2, OneLambda) and biotinylated goat anti-rabbit Fc (#111-066-046, JacksonImmunoResearch). Detection of biotinylated goat anti-rabbit Fc was achieved by using streptavidin-Alexa Fluor 647 (SA-AF647) (#016-600-084, JacksonImmunoResearch).

Human serum samples (n=119), both males and females, were purchased from BioIVT (BioIVT, Westbury, NY, USA) and used for quantification of Human Anti-Mouse Antibody (HAMA) and allo-specific antibody screening. Anonymized clinical study samples from highly HLA sensitized patients (n=8) and phase 1 healthy volunteers (n=11) were screened for HAMA and HLA allo-responsiveness against anti-CD19 CAR-Jurkat T-cells. Control sera for HLA allogeneic reactivity were purchased from OneLambda, including FlowPRA HLA Class I positive control serum (FL1-PC, OneLambda, West Hills, CA, USA), FlowPRA HLA Class II positive control serum (FL1-PC, OneLambda), and HLA negative control serum (LS-NC, LABScreen, OneLambda) or research samples donated from healthy donor.

### ELISA-based HAMA detection

The bridging ELISA kit "LEGEND MAX Human Anti-Mouse Ig (HAMA)" (Cat. No. 438307, Lot. No. B329842, BioLegend, San Diego, CA, USA) was used for detection of HAMA in human serum. In short, mouse IgG-precoated plates were washed and incubated with undiluted human serum samples (BioIVT), HAMA quality control, standard curve samples and mouse-IgG conjugate. The contents were discarded, and the plates washed with wash buffer. Substrate solution was added, and the plates were incubated in dark for 15 minutes at room temperature (RT). The reaction was terminated by addition of stop solution. The absorbance was measured at 450 and 570 nm within 30 minutes using a SpectraMax i3x spectrophotometer (SpectraMax i3x, Molecular Devices, San Jose, CA, USA). OD results were analyzed in Graph Pad Prism 9 (GraphPad Software, San Diego, CA, USA) using a 4-parameter logistics curve-fitting algorithm. Serum samples above 10 ng/ml HAMA were deemed HAMA positive.

### Flow cytometry screening for CAR-specific human sera

Anti-CD19 CAR-Jurkat or Jurkat wildtype T-cells (1x10E5/well) were washed in PBS and centrifuged at 300 g for 5 min. Cell pellets were resuspended in 50 µl selected human serum samples from the ELISA-based HAMA detection. The serum incubated cells were then washed and stained with a goat anti-human Fc-PE antibody (OneLambda) and analyzed by flow cytometry (CytoFLEX flow cytometer, # C02945, Beckman Coulter) in FL2 for mean fluorescence intensity (MFI) levels.

### Flow cytometry screening for CAR-T Jurkat allo-responsive human sera

Anti-CD19 CAR-Jurkat T-cells (1x10E5) were washed in PBS and centrifuged at 300 g for 5 min. Cell pellets were resuspended in 50 µl of indicated human serum samples. As controls FlowPRA Class I positive control serum (OneLambda), FlowPRA Class II positive control serum (OneLambda,) and HLA negative control serum (OneLambda) was used. Bound allo-antibodies were detected using a PE-conjugated goat anti-human IgG (LS-AB2, OneLambda). After a washing step the cells were resuspended in PBS and analyzed for MFI values using a flow cytometer (CytoFLEX).

### Polyclonal IgG binding to primary CAR-T

Primary human CAR T cells, including the anti-BCMA4 CAR T-cell (PM-CAR1037, ProMab Biotechnologies), anti-CD19 CAR T-cell (PM-CAR1007, ProMab Biotechnologies), and the Mock scFv control cells (PM-CAR1000, ProMab Biotechnologies) were thawed, washed, and incubated with rabbit anti-mouse IgG, F(ab')₂ specific (# 315-005-006, JacksonImmunoResearch) for 30 min. BCR expressing Daudi cells were stained as F(ab')₂-positive controls. Cells were then washed and incubated with biotinylated goat anti-rabbit Fc antibody and SA-AF647 (#016-600-084, JacksonImmunoResearch), consecutively. Similar approach was used to evaluate the binding of the polyclonal anti-mouse and anti-human IgG antibodies to the anti-CD19 CAR-Jurkat T-cells.

### Antibody-dependent cellular phagocytosis (ADCP) using FcγRI expressing effector cells

Anti-CD19 CAR-Jurkat T-cells were incubated with sera and tested for ADCP induction using a FcγRI Reporter Bioassay kit (#CS1781C01, Promega, Madison, WI, USA). In short, human serum samples (BioIVT, and anonymized sera from 06-study), HLA class I positive control serum (FL1-PC, OneLambda) and HLA negative control serum (#LS-NC, OneLambda) were incubated with or without 10 µg/mL imlifidase for 30 minutes at 37°C. Anti-CD19 CAR-Jurkat target T-cells (CARJ-ZP005, Creative Biolabs) (7500/well) were centrifuged, washed once in D-PBS, resuspended in the provided assay buffer (4% low IgG serum in RPMI-1640). Target cells were incubated with Imlifidase treated/non-treated antibodies and sera for 1 hour at 37°C. Following this, effector cells (75000/well), expressing FcyRI (Promega), were added to opsonized target cells, and incubated for 6 hours at 37°C. Finally, cells were incubated with Bio-Glo^{™} Luciferase Assay Reagent (Promega) at ambient temperature for 10 minutes before the luciferase activity was measured using a luminescence reader (SpectraMax i3x), where the integration time was set to 0.5 sec/well. Data was analyzed using GraphPad Prism 9.0 (GraphPad Software, San Diego, CA, USA). Plate background was calculated as average of three replicates including assay buffer, whereas no antibody control was calculated as average of duplicates containing only cells in presence/absence of imlifidase. The fold of induction (FoI) was calculated as follows, FoI = RLU (induced - background)/RLU (no antibody control - background).

### Antibody-dependent cell-mediated cytotoxicity (ADCC) using FcgRIIIa (V158) high affinity and FcgRIIIa (F158) low affinity expressing effector cells

Antibodies (rituximab, MabThera, Roche, Basel, Switzerland), rabbit anti-human IgG, F(ab')₂ specific (JacksonImmunoResearch), rabbit anti-mouse IgG, F(ab')₂ specific (JacksonImmunoResearch), and human serum (BioIVT and anonymized sera from highly HLA sensitized patients were incubated with or without 20 µg/ml imlifidase for 30 minutes at 37°C, and thereafter stored at 4°C overnight. Target cells (7500 cells/well), including anti-CD19 CAR-Jurkat (CARJ-ZP005, Creative Biolabs), Jurkat wt (EP-CL-0129, Elabscience, Houston, TX, USA) and Daudi (ACC78, DSMZ, Braunschweig, Germany) cells were centrifuged, washed once in D-PBS (GIBCO Life Technologies, Grand Island, NY, USA), resuspended in assay buffer (4% low IgG serum in RPMI-1640 (Promega, Madison, WI, USA)), and incubated with imlifidase treated/non-treated antibodies and serum for 1 hour at 37°C. Following this, 75000 effector cells, expressing either low (#G979A, Promega) or high affinity (#G701A, Promega) FcγRIIIa, were added to opsonized target cells, and incubated for 6 hours at 37°C. Finally, cells were incubated with Bio-Glo^{™} Luciferase Assay Reagent (Promega) at ambient temperature for 10 minutes before the luciferase activity was measured using a luminescence reader (SpectraMax i3x), where the integration time was set to 0.5 sec/well. Raw data was exported and analyzed using GraphPad Prism 9.0 (GraphPad Software). Plate background was calculated as average of tree replicates including assay buffer, whereas no antibody control was calculated as average of duplicates containing only cells in presence/absence of imlifidase. A HLA negative control, representing a serum sample from a healthy donor with no quantitative anti-HLA Class I and Class II antibodies, was also included. The fold of induction (FoI) was calculated as follows, FoI = RLU (induced - background)/RLU (no antibody control - background).

### Flow cytometry-based ADCP assay

ADCP target cells (anti-CD19 CAR-Jurkat T-cells, Jurkat wt T-cells and Daudi cells) were stained with calcein-AM (C3099, Invitrogen, Carlsbad, CA, USA) prior to incubation with imlifidase-treated (+/- 10 µg/mL) rabbit anti-human IgG, F(ab')₂ specific (#309-005-006, JacksonImmunoResearch) or anti-mouse IgG, F(ab')₂ specific (#315-005-006, JacksonImmunoResearch) antibodies at indicated concentrations. Alternatively, target cell pellets were resuspended in neat with imlifidase-treated (+/- 10 µg/mL) human serum samples (25 µl) for opsonization with HAMA or allogeneic IgG. The monocytic effector cell line, THP-1, was stained with CellTrace FarRed DDAO-SE (C34553, Molecular Probes, Eugene, OR, USA) before being added to the target cells and incubated for 90 min at 37°C. Phagocytosis was evaluated by flow cytometry (CytoFLEX flow cytometer, # C02945, Beckman Coulter). The amount of FL1 and FL4 double positive cells, reflecting phagocytized target cells by THP1 cells, were expressed as percentage of target cells.

### CD19-protein binding blocking experiment

Rabbit anti-mouse IgG, F(ab')₂ specific antibody (JacksonImmunoResearch Laboratories) and human sera (BioIVT) were incubated with or without 10 µg/ml imlifidase for 60 minutes at 37°C. IHAc (1mM) (I4386, Sigma-Aldrich, St. Louis, MO, USA) was added to all samples for 30 min to inactivate imlifidase during following incubation steps. Anti-CD19 CAR-Jurkat and Jurkat wt T-cells were incubated with the prepared serum and IgG samples for 60 min at RT. Recombinant human CD19-Fc chimeric protein, atto 647N conjugated (ATM9269, R&D systems, Minneapolis, MN, USA) was added to the cells and incubated for 45 min before analysed by flow cytometry (CytoFLEX flow cytometer, # C02945, Beckman Coulter).

### Results

### F(ab')₂-specific polyclonal antibodies bind specifically to CAR T-cell receptors

Primary human T-cells transfected with anti-CD19 or BCMA-specific chimeric antigen receptors to generate autologous CAR T-cells were used for identification of CAR-specific antibodies. When antigen-specific scFv-domains of the CARs originate from e.g. murine monoclonal antibodies they contain epitopes foreign to their recipient, even in the case of autologous CAR T-cell treatment. To investigate the effect of CAR-specific antibodies on CAR T-cells, different sources of CAR-specific antibodies were tested for binding. Polyclonal rabbit anti-mouse F(ab')₂ specific antibodies showed cross-reactivity to the anti-CD19 CAR T-cells (Figure 7A) and anti-BCMA-CAR T-cells (Figure 7B), while mock transfected T-cells (Figure 7C) remained unstained. B-cell receptor expressing Daudi cells (Figure 7D), having earlier been shown to be cross-reactive for the polyclonal rabbit anti-mouse F(ab')₂ detection reagent, were used for F(ab')₂-positive control staining. Staining of the human T-cell line Jurkat, transduced with anti-human CD19 CAR, where the scFv of the receptor is based on the murine mAb FMC63, demonstrated strong binding of the rabbit anti-mouse F(ab')₂ antibody. Some cross-reactivity was even observed using a rabbit anti-human F(ab')₂. These data confirm that various different receptor constructs against different targets can be bound by antibodies. Such binding may exert negative effects on adoptive cell transfer immunotherapies such as CAR T-cell treatment, so the antibodies were further used to investigate anti-CAR T-cell antibody- mediated effector functions and in how far these can be prevented by treatment with imlifidase.

### Identification of HAMA and CD19-CAR Jurkat T-cell allo-specific sera

One group of antibodies being potentially CAR-specific are human anti-mouse antibodies (HAMA). HAMA levels in human serum samples can be quantified with murine-IgG coated assay plates in a sandwich ELISA. A validated HAMA ELISA kit was used to screen human sera for HAMA (Figure 8A). A selection of identified HAMA-positive and - negative samples were tested for binding to anti-CD19 CAR-Jurkat T-cells. Jurkat wt T-cells were included to distinguish the binding of HAMA-specific IgG from HLA-allogeneic responses. It could be demonstrated that ELISA HAMA positive sera also bind specifically to anti-CD19 CAR T-cell receptors (Figure 8B). Depicted are sera screened by ELISA for HAMA (Figure 8C). These samples were then further screened by flow cytometry for binding of IgG. This allowed for the selection of HAMA- or anti-CD19 CAR T-cell alloreactive sera to further dissect the effect of these different IgG groups on anti-CD19 CAR-Jurkat T-cells. HAMA is believed to be induced in normal individuals from contact with murine antigens. The frequency and concentration of HAMA can be expected to be even higher in patients receiving murine mAb-based biologics, in some cases even leading to partial neutralization of these therapeutics. Higher levels of allogenic antibodies against anti-CD19 CAR- Jurkat T-cells can be detected in sera from HLA-sensitized transplantation patients compared to healthy individuals (Figure 8D). Five out of eight screened highly anti-HLA sensitized patients were positive and one patient highly anti HLA-sensitized towards anti-CD19 CAR-Jurkat T-cells. Two of the screened individuals did not have allo-antibodies against anti-CD19 CAR Jurkat cells.

These data demonstrate that antibodies capable of binding receptor constructs are detectable in human patients, including healthy individuals, and are increased in patients that have undergone a treatment that may increase HLA-sensitisation, such as a transplant.

Allogenic antibodies can not only be induced in organ transplantation but also be induced due to pregnancies and blood transfusions. Infusion of allogeneic cell therapies into patients might be an additional inducer of allogeneic antibodies, potentially posing a problem for allogenic CAR T-cell treatments.

### Polyclonal anti-F(ab')₂ antibodies opsonization of anti-CD19 CAR T-cells for ADCP is prevented by imlifidase treatment and imlifidase prevents ADCP-induction by allogeneic serum opsonized CD19-CAR Jurkat T-cells

Polyclonal anti-F(ab')₂ antibodies, specific for the CD19 scFv CAR domain, and HLA-specific antibodies directed against allogeneic anti-CD19 CAR-Jurkat T-cells, were tested for induction of antibody-mediated cell phagocytosis (ADCP) and the prevention thereof through treatment with the IgG-cleaving enzyme imlifidase (see Figure 9 and Figure 10). A flow cytometry based ADCP model was used with calcein-stained target cells and CellTrace FarRed labeled monocytic THP1 effector cells. Acquisition of cells by flow cytometry allowed discrimination of single and double positive cells i.e. phagocytosed cells. Anti-CD19 CAR- Jurkat target cells were opsonized with either rabbit anti-mouse F(ab')₂ (Figure 9A) or cross-reactive anti-human F(ab')₂ antibodies (Figure 9B). In both cases the ADCP of target cells was prevented by addition of imlifidase (10 µg/mL). There was no uptake of Jurkat wt cells, indicating that the phagocytosis was anti-CD19 CAR-dependent (Figure 9D, E). BCR-positive Daudi target cells were used as positive control for F(ab')₂ antibody mediated ADCP (Figure 9C, F). These data demonstate that receptor-specific antibodies can induce ADCP against T-cells, which is expected to negatively affect cell therapies. The data also demonstate that treatment with imlifidase is effective to prevent the ADCP. In addition, the opsonization and induction of ADCP by human sera, sensitized against allogeneic Jurkat CAR T-cell, were tested using a bioluminescence FcyRI reporter assay (#CS1781C01, Promega, #CS1781C01). Serum from normal individuals (Figure 10A) and highly anti-HLA sensitized patients (Figure 10B) were able to opsonize anti-CD19 CAR-Jurkat T-cells and induce ADCP. The ADCP induction by serum IgG against allogeneic cells could be reduced through treatment with imlifidase. Therefore, these data confirm that the antibodies in serum from both normal individuals and sensitized patients induce ADCP against adoptive cell transfer immunotherapy cells, but this can be reduced by imlifidase treatment.

### FcγRIIIa allele (V158) and allele (F158) anti-CD19 CAR-specific antibody-induced ADCC (V158) is prevented by imlifidase treatment.

Antibody-dependent cell cytotoxicity (ADCC) is triggered through the engagement of FcyRIIIa (CD16a) on effector cells by IgG-opsonized target cells. Two main CD16a alleles exist in the general population, a high affinity variant having a valine at position 158 (V158), and a low affinity variant with phenylalanine at position 158 (F158). These alleles were introduced into effector cells of an ADCC bioluminescence assays (Promega). Anti-CD19 CAR-Jurkat T-cells were incubated with either rabbit anti-mouse (Figure 11A) or anti-human (Figure 11B) F(ab')₂ antibodies, with and without imlifidase (20 µg/mL) treatment. The opsonized target cells were incubated with high affinity FcyRIIIa (V158) effector cells. ADCC was strongly induced by the anti-mouse F(ab')₂ antibody opsonized target cells. The induction of ADCC was fully abolished in the imlifidase treated samples (Figure 11A). Polyclonal anti-human F(ab')₂ induced a weaker stimulating effect but could trigger ADCC at 100 µg/mL (Figure 11B), an effect that was not seen in the negative control, Jurkat wt cells (Figure 11C). Daudi cells opsonized with rituximab (Figure 11D) or anti-human F(ab')₂ (Figure 11E) were used as positive control. Also, imlifidase treatment of opsonized Daudi target cells prevented induction of ADCC (Figure 11 D, E).

Similar results were seen using the low affinity FcyRIIIa ADCC bioluminescence reporter assay (Figure 12). Anti-mouse F(ab')₂ treated anti-CD19 CAR-Jurkat T-cells induced ADCC signaling, which could be prevented by treatment with imlifidase (Figure 12A). On the other hand, polyclonal anti-human F(ab')₂ antibodies could not trigger ADCC signaling even at the highest (100 µg/mL) antibody concentration (Figure 12B). Daudi cells incubated with rituximab (Figure 12D) or anti-human F(ab')₂ (Figure. 12E) induced low affinity FcyRIIIa ADCC, an effect that was prevented by imlifidase treatment.

These data further demonstrate that receptor-specific antibodies can mediate deleterious effects against adoptive cell transfer immunotherapy cells, in particular ADCC, which is expected to negatively affect cell therapies. The data also demonstate that treatment with imlifidase is effective to prevent the ADCC.

### ADCC-induction by HAMA-opsonized anti-CD19 CAR-Jurkat T-cells can be prevented with imlifidase treatment

ELISA-HAMA negative (164) and positive sera (184, 187, 208, 250) treated with or without 20 µg/mL imlifidase were incubated with anti-CD19 CAR-Jurkat T-cells before adding the opsonized target cells to FcyRIIIa high affinity allele transfected effector cells (Promega). The bioluminescent signals from two HAMA positive sera (184, 250) were reduced in presence of imlifidase (Figure 13). This suggests that a fraction of the mouse-IgG binding HAMA antibodies in human serum bind to the anti-CD19 CAR in a way that allows triggering of CD16 FcγRIIIa of effector cells. Therefore, these data further demonstate that the antibodies in human sera mediate deleterious effects against adoptive cell transfer immunotherapy cells, but this can be reduced by imlifidase treatment.

### CD19-protein binding to serum-exposed anti-CD19 CAR T-cell can be improved by imlifidase treatment

For a successful CAR T-cell therapy, the specific interaction of their chimeric antigen receptor with its target protein is a required step. The binding of serum IgG to the anti-CD19 CAR could interfere with its cognate engagement with e.g. CD19 on target cells. HAMA positive and negative serum samples (Figure 14), identified by ELISA, were tested for interference of the binding of recombinant atto-647N-labeled human CD19 protein with anti-CD19 CAR-Jurkat T-cells. The digestion of serum IgG by imlifidase resulted, in most samples, to an increased median FI signal derived from anti-CD19 CAR-Jurkat T-cells, suggesting an increased binding of atto-647N-labeled CD19-protein. This suggests that the interaction of adoptive cell immunotherapies, such as anti-CD19 CAR T-cells, with their target protein can be increased through the mere removal of the IgG-Fc part by imlifidase treatment, which could reduce steric hindrance. The *in vitro* effect of imlifidase could be even stronger *in vivo* since blocking F(ab')2 fragments will have a shorter half-life than their intact IgG equivalent.

## Claims

1. A protein that has IgG cysteine protease or IgG endoglycosidase activity for use in medicine, wherein the protein that has IgG cysteine protease or IgG endoglycosidase activity is used in combination with an adoptive cell transfer immunotherapy.

2. A protein that has IgG cysteine protease or IgG endoglycosidase activity for use in a method for treating cancer, wherein the protein that has IgG cysteine protease or IgG endoglycosidase activity is used in combination with an adoptive cell transfer immunotherapy.

3. A protein that has IgG cysteine protease or IgG endoglycosidase activity for use in a method for treating an autoimmune condition, wherein the protein that has IgG cysteine protease or IgG endoglycosidase activity is used in combination with an adoptive cell transfer immunotherapy.

4. A protein that has IgG cysteine protease or IgG endoglycosidase activity for use in treating a condition mediated by B-cells producing deleterious antibodies, wherein the protein that has IgG cysteine protease or IgG endoglycosidase activity is used in combination with an adoptive cell transfer immunotherapy.

5. A protein that has IgG cysteine protease or IgG endoglycosidase activity for use in a method for treating an infection, wherein the protein is used in combination with an adoptive cell transfer immunotherapy.

6. The protein of any preceding claim for the use of any preceding claim, wherein the protein that has IgG cysteine protease or IgG endoglycosidase activity is administered prior to administration of the adoptive cell transfer immunotherapy, or wherein the protein that has IgG cysteine protease or IgG endoglycosidase activity is administered after administration of the adoptive cell transfer immunotherapy.

7. The protein that has IgG cysteine protease or IgG endoglycosidase activity of any one of claims 2 to 5 for the use of any one of claims 2 to 5, wherein the method comprises administering the protein to a patient that previously received and/or is scheduled to receive an adoptive cell transfer immunotherapy.

8. An adoptive cell transfer immunotherapy for use in a method for treating cancer, an autoimmune condition, a condition mediated by B-cells producing deleterious antibodies or an infection, wherein the method comprises administering the adoptive cell transfer immunotherapy to a patient that previously received and/or is scheduled to receive a protein that has IgG cysteine protease or IgG endoglycosidase activity.

9. The protein or the adoptive cell transfer immunotherapy of any preceding claim for the use of any preceding claim, wherein the adoptive cell transfer immunotherapy comprises administration of T-cells, monocytes, macrophages, natural killer cells or dendritic cells expressing a chimeric antigen receptor or a T-cell receptor.

10. The protein or the adoptive cell transfer immunotherapy of any preceding claim for the use of any preceding claim, wherein:
(i) the protein having IgG cysteine protease activity is an IgG cysteine protease from a *Streptococcus* bacterium such as *Streptococcus pyogenes,* optionally wherein said protein is IdeS or IdeZ; or
(ii) the protein having IgG endoglycosidase activity is an IgG endoglycosidase from a *Streptococcus* bacterium, such as *Streptococcus pyogenes, Streptococcus equi* or *Streptococcus zooepidemicus,* or from *Corynebacterium pseudotuberculosis, Enterococcus faecalis,* or *Elizabethkingia meningoseptica,* optionally wherein said protein is EndoS, CP40, EndoE, or EndoF₂.

11. The protein or the adoptive cell transfer immunotherapy of claim 10 for the use of claim 10, wherein
(i) the protein having IgG cysteine protease activity is a polypeptide which comprises or consists of the amino acid sequence of SEQ ID NO: 2, 4 or 5, or a fragment or variant thereof which has IgG cysteine protease activity; or
(ii) the protein having IgG endoglycosidase activity is a polypeptide which comprises or consists of the amino acid sequence of SEQ ID NO: 90, or a fragment or variant thereof which has IgG endoglycosidase activity.

12. The protein or the adoptive cell transfer immunotherapy of claim 10 for the use of claim 10, wherein
(i) the protein having IgG cysteine protease activity is a polypeptide having a sequence that is at least 80% identical to SEQ ID NO: 2, 4 or 5, such as at least 85%, 90%, 95% or 99% identical, or wherein said IgG cysteine protease comprises or consists of the sequence of any one of SEQ ID NOs: 6 to 25 and 55 to 69, optionally wherein said sequence includes an additional methionine at the N terminus and/or a histidine tag at the C terminus; or
(ii) the protein having IgG endoglycosidase activity is a polypeptide having a sequence that is at least 80% identical to SEQ ID NO: 90, such as at least 85%, 90%, 95% or 99% identical.

13. The protein or the adoptive cell transfer immunotherapy of any preceding claim for the use of any preceding claim, wherein the protein that has IgG cysteine protease or IgG endoglycosidase activity is administered prior to, subsequent to or concurrently with the adoptive cell transfer immunotherapy.

14. The protein or the adoptive cell transfer immunotherapy of any preceding claim for the use of any preceding claim, wherein the protein that has IgG cysteine protease or IgG endoglycosidase activity is formulated in a first pharmaceutical compositon and the adoptive cell transfer immunotherapy is formulated in a second pharmacuetical composition.

15. The protein or the adoptive cell transfer immunotherapy of any preceding claim for the use of any preceding claim, wherein
(i) the protein that has IgG cysteine protease or IgG endoglycosidase activity is synthesised using Fmoc solid phase chemistry, Boc solid phase chemistry or by solution phase peptide synthesis; or
(ii) the protein that has IgG cysteine protease or IgG endoglycosidase activity is synthesised by transforming a bacterial cell with a nucleic acid molecule or vector which encodes the protein that has IgG cysteine protease or IgG endoglycosidase activity.

## Patentansprüche

1. Protein, das IgG-Cysteinprotease- oder IgG-Endoglykosidase-Aktivität aufweist, für die Verwendung in der Medizin, wobei das Protein, das IgG-Cysteinprotease- oder IgG-Endoglykosidase-Aktivität aufweist, in Kombination mit einer adoptiven Zelltransferimmuntherapie verwendet wird.

2. Protein, das IgG-Cysteinprotease- oder IgG-Endoglykosidase-Aktivität aufweist, für die Verwendung in einem Verfahren zum Behandeln von Krebs, wobei das Protein, das IgG-Cysteinprotease- oder IgG-Endoglykosidase-Aktivität aufweist, in Kombination mit einer adoptiven Zelltransferimmuntherapie verwendet wird.

3. Protein, das IgG-Cysteinprotease- oder IgG-Endoglykosidase-Aktivität aufweist, für die Verwendung in einem Verfahren zum Behandeln einer Autoimmunerkrankung, wobei das Protein, das IgG-Cysteinprotease- oder IgG-Endoglykosidase-Aktivität aufweist, in Kombination mit einer adoptiven Zelltransferimmuntherapie verwendet wird.

4. Protein, das IgG-Cysteinprotease- oder IgG-Endoglykosidase-Aktivität aufweist, für die Verwendung zum Behandeln eines durch B-Zellen vermittelten Zustands, der schädliche Antikörper produziert, wobei das Protein, das IgG-Cysteinprotease- oder IgG-Endoglykosidase-Aktivität aufweist, in Kombination mit einer adoptiven Zelltransferimmuntherapie verwendet wird.

5. Protein, das IgG-Cysteinprotease- oder IgG-Endoglykosidase-Aktivität aufweist, für die Verwendung in einem Verfahren zum Behandeln einer Infektion, wobei das Protein in Kombination mit einer adoptiven Zelltransferimmuntherapie verwendet wird.

6. Protein nach einem der vorhergehenden Ansprüche für die Verwendung nach einem der vorhergehenden Ansprüche, wobei das Protein, das IgG-Cysteinprotease- oder IgG-Endoglykosidase-Aktivität aufweist, vor der Verabreichung der adoptiven Zelltransferimmuntherapie verabreicht wird oder wobei das Protein, das IgG-Cysteinprotease- oder IgG-Endoglykosidase-Aktivität aufweist, nach der Verabreichung der adoptiven Zelltransferimmuntherapie verabreicht wird.

7. Protein, das IgG-Cysteinprotease- oder IgG-Endoglykosidase-Aktivität aufweist, nach einem der Ansprüche 2 bis 5, für die Verwendung nach einem der Ansprüche 2 bis 5, wobei das Verfahren das Verabreichen des Proteins an einen Patienten umfasst, der zuvor eine adoptive Zelltransferimmuntherapie erhalten hat und/oder diese erhalten soll.

8. Adoptive Zelltransferimmuntherapie für die Verwendung in einem Verfahren zum Behandeln von Krebs, einer Autoimmunerkrankung, eines durch B-Zellen vermittelten Zustands, der schädliche Antikörper produziert, oder einer Infektion, wobei das Verfahren das Verabreichen der adoptiven Zelltransferimmuntherapie an einen Patienten umfasst, der zuvor ein Protein erhalten hat und/oder erhalten soll, das IgG-Cysteinprotease- oder IgG-Endoglykosidase-Aktivität aufweist.

9. Protein oder adoptive Zelltransferimmuntherapie nach einem der vorstehenden Ansprüche für die Verwendung nach einem der vorstehenden Ansprüche, wobei die adoptive Zelltransferimmuntherapie die Verabreichung von T-Zellen, Monozyten, Makrophagen, natürlichen Killerzellen oder dendritischen Zellen umfasst, die einen chimären Antigenrezeptor oder einen T-Zell-Rezeptor exprimieren.

10. Protein oder adoptive Zelltransferimmuntherapie nach einem der vorstehenden Ansprüche für die Verwendung nach einem der vorstehenden Ansprüche, wobei:
(i) das Protein mit IgG-Cysteinproteaseaktivität eine IgG-Cysteinprotease aus einem *Streptococcus-Bakterium* wie *Streptococcus pyogenes* ist, wobei das Protein optional IdeS oder IdeZ ist; oder
(ii) das Protein mit IgG-Endoglykosidaseaktivität eine IgG-Endoglykosidase aus einem *Streptococcus*-Bakterium, wie *Streptococcus pyogenes, Streptococcus equi* oder *Streptococcus zooepidemicus,* oder aus *Corynebacterium pseudotuberculosis, Enterococcus faecalis* oder *Elizabethkingia meningoseptica,* wobei das Protein optional EndoS, CP40, EndoE oder EndoF₂ ist.

11. Protein oder adoptive Zelltransferimmuntherapie nach Anspruch 10 für die Verwendung nach Anspruch 10, wobei
(i) das Protein mit IgG-Cysteinproteaseaktivität ein Polypeptid, das die Aminosäuresequenz von SEQ ID NO: 2, 4 oder 5 umfasst oder daraus besteht, oder ein Fragment oder eine Variante davon ist, das/die IgG-Cysteinproteaseaktivität aufweist; oder
(ii) das Protein mit IgG-Endoglykosidaseaktivität ein Polypeptid, das die Aminosäuresequenz von SEQ ID NO: 90 umfasst oder daraus besteht, oder ein Fragment oder eine Variante davon ist, das/die IgG-Endoglykosidaseaktivität aufweist.

12. Protein oder adoptive Zelltransferimmuntherapie nach Anspruch 10 für die Verwendung nach Anspruch 10, wobei
(i) das Protein mit IgG-Cysteinproteaseaktivität ein Polypeptid mit einer Sequenz ist, die wenigstens 80 % mit SEQ ID NO: 2, 4 oder 5, wie weniger als 85 %, 90 %, 95 % oder 99 % identisch ist, oder wobei die IgG-Cysteinprotease die Sequenz einer der SEQ ID NO: 6 bis 25 und 55 bis 69 umfasst oder daraus besteht, wobei die Sequenz optional ein zusätzliches Methionin an dem N-Terminus und/oder ein Histidin-Tag an dem C-Terminus beinhaltet; oder
(ii) das Protein mit IgG-Endoglykosidaseaktivität ein Polypeptid mit einer Sequenz ist, die wenigstens 80 % mit SEQ ID NO: 90 identisch ist, wie wenigstens 85 %, 90 %, 95 % oder 99 % identisch.

13. Protein oder adoptive Zelltransferimmuntherapie nach einem der vorstehenden Ansprüche für die Verwendung nach einem der vorhergehenden Ansprüche, wobei das Protein, das IgG-Cysteinprotease- oder IgG-Endoglykosidase-Aktivität aufweist, vor, nach oder gleichzeitig mit der adoptiven Zelltransferimmuntherapie verabreicht wird.

14. Protein oder adoptive Zelltransferimmuntherapie nach einem der vorhergehenden Ansprüche für die Verwendung nach einem der vorstehenden Ansprüche, wobei das Protein, das IgG-Cysteinprotease- oder IgG-Endoglykosidase-Aktivität aufweist, in einer ersten pharmazeutischen Zusammensetzung formuliert ist und die adoptive Zelltransferimmuntherapie in einer zweiten pharmazeutischen Zusammensetzung formuliert ist.

15. Protein oder adoptive Zelltransferimmuntherapie nach einem der vorstehenden Ansprüche für die Verwendung nach einem der vorstehenden Ansprüche, wobei
(i) das Protein, das eine IgG-Cysteinprotease- oder IgG-Endoglykosidase-Aktivität aufweist, unter Verwendung von Fmoc-Festphasenchemie, Boc-Festphasenchemie oder durch Lösungsphasenpeptidsynthese synthetisiert wird; oder
(ii) das Protein, das IgG-Cysteinprotease- oder IgG-Endoglykosidase-Aktivität aufweist, durch Transformatieren einer Bakterienzelle mit einem Nukleinsäuremolekül oder Vektor synthetisiert wird, das/der für das Protein codiert, das IgG-Cysteinprotease- oder IgG-Endoglykosidase-Aktivität aufweist.

## Revendications

1. Protéine qui a une activité d'IgG cystéine protéase ou d'IgG endoglycosidase pour une utilisation en médecine, dans laquelle la protéine qui a une activité d'IgG cystéine protéase ou d'IgG endoglycosidase est utilisée en combinaison avec une immunothérapie par transfert adoptif de cellules.

2. Protéine qui a une activité d'IgG cystéine protéase ou d'IgG endoglycosidase pour une utilisation dans un procédé de traitement du cancer, dans laquelle la protéine qui a une activité d'IgG cystéine protéase ou d'IgG endoglycosidase est utilisée en combinaison avec une immunothérapie par transfert adoptif de cellules.

3. Protéine qui a une activité d'IgG cystéine protéase ou d'IgG endoglycosidase pour une utilisation dans un procédé de traitement d'une affection auto-immune, dans laquelle la protéine qui a une activité d'IgG cystéine protéase ou d'IgG endoglycosidase est utilisée en combinaison avec une immunothérapie par transfert adoptif de cellules.

4. Protéine qui a une activité d'IgG cystéine protéase ou d'IgG endoglycosidase pour une utilisation dans un procédé de traitement d'une affection médiée par la production par les lymphocytes B d'anticorps délétères, dans laquelle la protéine qui a une activité d'IgG cystéine protéase ou d'IgG endoglycosidase est utilisée en combinaison avec une immunothérapie par transfert adoptif de cellules.

5. Protéine qui a une activité d'IgG cystéine protéase ou d'IgG endoglycosidase pour une utilisation dans un procédé de traitement d'une infection, dans laquelle la protéine est utilisée en combinaison avec une immunothérapie par transfert adoptif de cellules.

6. Protéine selon l'une quelconque des revendications précédentes pour l'utilisation de l'une quelconque des revendications précédentes, dans laquelle la protéine qui a une activité d'IgG cystéine protéase ou d'IgG endoglycosidase est administrée avant l'administration de l'immunothérapie par transfert adoptif de cellules, ou dans laquelle la protéine qui a une activité d'IgG cystéine protéase ou d'IgG endoglycosidase est administrée après l'administration de l'immunothérapie par transfert adoptif de cellules.

7. Protéine qui a une activité d'IgG cystéine protéase ou d'IgG endoglycosidase selon l'une quelconque des revendications 2 à 5 pour l'utilisation de l'une quelconque des revendications 2 à 5, dans laquelle le procédé comprend l'administration de la protéine à un patient qui a précédemment reçu et/ou pour lequel il est prévu de recevoir une immunothérapie par transfert adoptif de cellules.

8. Immunothérapie par transfert adoptif de cellules pour une utilisation dans un procédé de traitement du cancer, d'une affection auto-immune, d'une affection médiée par la production par les lymphocytes B d'anticorps délétères ou d'une infection, dans laquelle le procédé comprend l'administration de l'immunothérapie par transfert adoptif de cellules à un patient qui a précédemment reçu et/ou pour lequel il est prévu de recevoir une protéine qui a une activité d'IgG cystéine protéase ou d'IgG endoglycosidase.

9. Protéine ou immunothérapie par transfert adoptif de cellules selon l'une quelconque des revendications précédentes pour l'utilisation de l'une quelconque des revendications précédentes, dans laquelle l'immunothérapie par transfert adoptif de cellules comprend l'administration de lymphocytes T, de monocytes, de macrophages, de cellules tueuses naturelles ou de cellules dendritiques exprimant un récepteur d'antigène chimérique ou un récepteur de lymphocytes T.

10. Protéine ou immunothérapie par transfert adoptif de cellules selon l'une quelconque des revendications précédentes pour l'utilisation de l'une quelconque des revendications précédentes, dans laquelle :
(i) la protéine ayant une activité d'IgG cystéine protéase est une IgG cystéine protéase issue d'une bactérie *Streptococcus* telle que *Streptococcus pyogenes,* éventuellement dans laquelle ladite protéine est IdeS ou IdeZ ; ou
(ii) la protéine ayant une activité d'IgG endoglycosidase est une IgG endoglycosidase issue d'une bactérie *Streptococcus,* telle que *Streptococcus pyogenes, Streptococcus equi* ou *Streptococcus zooepidemicus,* ou issue de *Corynebacterium pseudotuberculosis, Enterococcus faecalis,* ou *Elizabethkingia meningoseptica,* éventuellement dans laquelle ladite protéine est EndoS, CP40, EndoE, ou EndoF₂.

11. Protéine ou immunothérapie par transfert adoptif de cellules selon la revendication 10 pour l'utilisation de la revendication 10, dans laquelle
(i) la protéine ayant une activité d'IgG cystéine protéase est un polypeptide qui comprend ou est constitué par la séquence d'acides aminés de SEQ ID N° : 2, 4 ou 5, ou un fragment ou variant de celui-ci qui a une activité d'IgG cystéine protéase ; ou
(ii) la protéine ayant une activité d'IgG endoglycosidase est un polypeptide qui comprend ou est constitué par la séquence d'acides aminés de SEQ ID N° : 90, ou un fragment ou variant de celui-ci qui a une activité d'IgG endoglycosidase.

12. Protéine ou immunothérapie par transfert adoptif de cellules selon la revendication 10 pour l'utilisation de la revendication 10, dans laquelle
(i) la protéine ayant une activité d'IgG cystéine protéase est un polypeptide ayant une séquence qui est identique à au moins 80 % à SEQ ID N° : 2, 4 ou 5, telle qu'identique à au moins 85 %, 90 %, 95 % ou 99 %, ou dans laquelle ladite IgG cystéine protéase comprend ou est constituée par la séquence de l'une quelconque parmi SEQ ID N° : 6 à 25 et 55 à 69, éventuellement dans laquelle ladite séquence comporte une méthionine supplémentaire à l'extrémité N-terminale et/ou un étiquette histidine à l'extrémité C-terminale ; ou
(ii) la protéine ayant une activité d'IgG endoglycosidase est un polypeptide ayant une séquence qui est identique à au moins 80 % à SEQ ID N° : 90, telle qu'identique à au moins 85 %, 90 %, 95 % ou 99 %.

13. Protéine ou immunothérapie par transfert adoptif de cellules selon l'une quelconque des revendications précédentes pour l'utilisation de l'une quelconque des revendications précédentes, dans laquelle la protéine qui a une activité d'IgG cystéine protéase ou d'IgG endoglycosidase est administrée antérieurement, ultérieurement ou simultanément à l'immunothérapie par transfert adoptif de cellules.

14. Protéine ou immunothérapie par transfert adoptif de cellules selon l'une quelconque des revendications précédentes pour l'utilisation de l'une quelconque des revendications précédentes, dans laquelle la protéine qui a une activité d'IgG cystéine protéase ou d'IgG endoglycosidase est formulée dans une première composition pharmaceutique et l'immunothérapie par transfert adoptif de cellules est formulée dans une deuxième composition pharmaceutique.

15. Protéine ou immunothérapie par transfert adoptif de cellules selon l'une quelconque des revendications précédentes pour l'utilisation de l'une quelconque des revendications précédentes, dans laquelle
(i) la protéine qui a une activité d'IgG cystéine protéase ou d'IgG endoglycosidase est synthétisée à l'aide de la chimie en phase solide Fmoc, de la chimie en phase solide Boc ou par synthèse peptidique en phase de solution ; ou
(ii) la protéine qui a une activité d'IgG cystéine protéase ou d'IgG endoglycosidase est synthétisée par transformation d'une cellule bactérienne avec une molécule ou un vecteur d'acide nucléique qui code pour la protéine qui a une activité d'IgG cystéine protéase ou d'IgG endoglycosidase.
